(19) **Europäisches Patentamt / European Patent Office / Office européen des brevets**

(11) **EP 4 238 984 A1**

(12) **EUROPEAN PATENT APPLICATION**
published in accordance with Art. 153(4) EPC

(43) Date of publication:
**06.09.2023 Bulletin 2023/36**

(21) Application number: **21886968.3**

(22) Date of filing: **01.11.2021**

(51) International Patent Classification (IPC):
*C07K 14/475* (2006.01)    *C07K 16/28* (2006.01)
*C07K 16/18* (2006.01)    *A61P 35/00* (2006.01)
*A61K 38/00* (2006.01)

(52) Cooperative Patent Classification (CPC):
**A61K 38/00; A61P 35/00; C07K 14/475;**
**C07K 16/18; C07K 16/28**

(86) International application number:
**PCT/KR2021/015602**

(87) International publication number:
**WO 2022/092974 (05.05.2022 Gazette 2022/18)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **30.10.2020 KR 20200143660**

(71) Applicant: **Cellemedy Co., Ltd**
**Seongnam-si, Gyeonggi-do 13486 (KR)**

(72) Inventors:
• **LEE, Jee Won**
**Seoul 06707 (KR)**

• **LEE, Bo Ram**
**Seoul 02476 (KR)**
• **YOON, Chul Joo**
**Seoul 06599 (KR)**

(74) Representative: **dompatent von Kreisler Selting**
**Werner -**
**Partnerschaft von Patent- und Rechtsanwälten**
**mbB**
**Deichmannhaus am Dom**
**Bahnhofsvorplatz 1**
**50667 Köln (DE)**

Remarks:
The complete document including Reference
Table(s) and the Sequence Listing(s) can be
downloaded from the EPO website

(54) **ANTIBODY-LIKE PROTEIN AND USE THEREOF**

(57)    The present invention relates to an antibody-like protein and use thereof, and more specifically, to an antibody-like protein and use thereof, wherein the antibody-like protein is formed by self-assembly of a plurality of ferritin monomers including at least CDR-ferritin monomers having a complementarity-determining region of an antibody fused thereto whereby the antibody-like protein has the complementarity-determining regions present at a high density on the surface or outside thereof so that even some of the complementarity-determining regions, such as HCDR3, etc., can bind to an antigen at an affinity level similar to that of the antibody, and the antibody-like protein possesses a structural feature advantageous for binding simultaneously to a plurality of antigens, and as such, has remarkably improved binding avidity, compared to the antibody. The antibody-like protein of the present invention is an antibody substitute that can be utilized in antibody-based uses and fields.

[FIG. 9]

**Description**

[Technical Field]

The present invention relates to a protein with a novel structure and features, and use thereof

[Background Art]

A target-oriented drug delivery system refers to a technology designed to selectively deliver a drug to a treatment site without exposing a healthy tissue to the drug, and show an excellent therapeutic effect with only a small amount of drug. When using the target-oriented drug delivery system, drug treatment effects may be maximized by concentrating the drug on a specific part of the body with a disease, and side effects caused by highly toxic drugs such as an anticancer drug may be minimized.

In almost all cases, signal peptides and antibodies are representative substances imparting target orientation. Antibodies are proteins produced by immune responses of vertebrates, and are immune proteins that specifically recognize and bind to a specific site of an antigen to inactivate or eliminate the action of the antigen. The antibody has two heavy chains and two light chains, and in the case of the most commonly used G-class antibody (immunoglobulin G), it basically forms a Y-shape. The variable regions of the light and heavy chains are combined to form an antigen binding site. Since such an antibody has two antigen-binding sites, one antibody can bind with only two antigenic epitopes. In addition, the portion occupied by the antigen-binding site corresponds to only a portion compared to the overall size of the antibody, and when using the antibody, there is a limitation in that other foreign sequences except for the antigen-binding site that may induce an antigen-antibody reaction are introduced into the administration body together.

Ferritin is a protein that stores iron and is widely present in prokaryotes and eukaryotes. Ferritin has a molecular weight of about 500,000 Da and includes heavy chains and light chains. Ferritin is a protein in which 24 monomers (a single monomer or heterogeneous monomer composed of either a heavy chain or a light chain) are gathered to form a huge spherical tertiary structure. In the case of human ferritin, it has an outer diameter of about 12 nm and an inner diameter of about 8 nm.

The ferritin structure can be modified using an appropriate functional group by a selective method, and has an advantage of imparting various physicochemical properties required. For example, a ferritin monomer fused with an antigenic epitope is self-assembled to form a ferritin protein, and the ferritin protein may serve to present an antigen to dendritic cells. However, a case in which the complementarity determining region of an antibody is fused to the ferritin monomer has not yet been reported.

[Summary of Invention]

[Problems to be Solved by Invention]

An object of the present invention is to provide an antibody-like protein having excellent antigen-avidity.

Another object of the present invention is to provide an antibody-like protein that does not induce an unnecessary immune response by using the entire antibody.

In addition, another object of the present invention is to provide an antibody-like protein that is easy to produce and store.

Further, another object of the present invention is to provide a pharmaceutical composition for treating or preventing a disease, a composition for diagnosing a disease, and a method for detecting an antigen using the antibody-like protein.

[Means for Solving Problems]

To achieve the above objects, according to an aspect of the present invention, there is provided an antibody-like protein including self-assembly of a plurality of ferritin monomers, in which at least a CDR-ferritin monomer fused with a complementarity determining region is included.

In the antibody-like protein of the present invention, the complementarity determining region may be any one selected from the group consisting of HCDR1, HCDR2, HCDR3, LCDR1, LCDR2, LCDR3, and conservative sequence variants thereof.

In the antibody-like protein of the present invention, the complementarity determining region may be exposed to a surface or outside of the protein for binding to an antigen.

In the antibody-like protein of the present invention, the complementarity determining region may have a length of 25aa or less.

In the antibody-like protein of the present invention, the CDR-ferritin monomer may be characterized in that the complementarity determining region is fused to a human ferritin heavy chain monomer.

In the antibody-like protein of the present invention, the complementarity determining region may be fused to any one selected from the group consisting of the inside of α-helix, between adjacent α-helices, N-terminus, C-terminus, A-B loop, B-C loop, C-D loop, D-E loop, between N-terminus and A helix, and between E helix and C-terminus of the ferritin monomer.

In the antibody-like protein of the present invention, the CDR-ferritin monomer may be characterized in that a plurality of complementarity determining regions of the antibody are fused to a single ferritin monomer.

In the antibody-like protein of the present invention, the CDR-ferritin monomer may be characterized in that at least one heavy chain complementarity determining region selected from the group consisting of HCDR1, HCDR2, HCDR3 and conservative sequence variants thereof of the antibody is fused to a single ferritin monomer.

In the antibody-like protein of the present invention, the CDR-ferritin monomer may be characterized in that at least one light chain complementarity determining region selected from the group consisting of LCDR1, LCDR2, LCDR3 and conservative sequence variants thereof of the antibody is fused to a single ferritin monomer.

In the antibody-like protein of the present invention, the CDR-ferritin monomer may include a heavy chain CDR-ferritin monomer to which at least one heavy chain complementarity determining region selected from the group consisting of HCDR1, HCDR2, HCDR3 and conservative sequence variants thereof of the antibody is fused, and a light chain CDR-ferritin monomer to which at least one light chain complementarity determining region selected from the group consisting of LCDR1, LCDR2, LCDR3 and conservative sequence variants thereof of the antibody is fused.

In the antibody-like protein of the present invention, the complementarity determining region may be fused to the DE loop or C-terminus of the CDR-ferritin monomer to form a four-fold symmetric structure.

In the antibody-like protein of the present invention, the complementarity determining region may be fused to the N-terminus, AB loop or BC loop of the CDR-ferritin monomer to form a two-fold symmetric structure.

In the antibody-like protein of the present invention, the complementarity determining region may be fused to the CD loop of the CDR-ferritin monomer to form a three-fold symmetric structure.

In the antibody-like protein of the present invention, the complementarity determining regions may be disposed to be spaced apart from each other by a distance of 0.7 to 7 nm.

In the antibody-like protein of the present invention, the CDR-ferritin monomer may be characterized in that each complementarity determining region of two or more antibodies is fused to a single ferritin monomer.

In the antibody-like protein of the present invention, the CDR-ferritin monomer may include a first CDR-ferritin monomer to which at least one complementarity determining region of a first antibody is fused, and a second CDR-ferritin monomer to which at least one complementarity determining region of a second antibody is fused.

In the antibody-like protein of the present invention, the first CDR-ferritin monomer may include a first heavy chain CDR-ferritin monomer to which at least one heavy chain complementarity determining region selected from the group consisting of HCDR1, HCDR2, HCDR3 and conservative sequence variants thereof of the first antibody is fused, and a first light chain CDR-ferritin monomer to which at least one light chain complementarity determining region selected from the group consisting of LCDR1, LCDR2, LCDR3 and conservative sequence variants thereof of the first antibody is fused.

In the antibody-like protein of the present invention, he second CDR-ferritin monomer may include a second heavy chain CDR-ferritin monomer to which at least one heavy chain complementarity determining region selected from the group consisting of HCDR1, HCDR2, HCDR3 and conservative sequence variants thereof of the second antibody is fused, and a second light chain CDR-ferritin monomer to which at least one light chain complementarity determining region selected from the group consisting of LCDR1, LCDR2, LCDR3 and conservative sequence variants thereof of the second antibody is fused.

In the antibody-like protein of the present invention, the first CDR-ferritin monomer and the second CDR-ferritin monomer may be included in a ratio of 1:1 to 1:5.

In the antibody-like protein of the present invention, the CDR-ferritin monomer may include at least a first CDR-ferritin monomer fused with a plurality of complementarity determining regions of a first antibody group including a plurality of different antibodies, and a second CDR-ferritin monomer fused with a plurality of complementarity determining regions of a second antibody group including a plurality of different antibodies.

In the antibody-like protein of the present invention, the plurality of ferritin monomers may include at least a ferritin monomer to which the complementarity determining region is not fused.

In the antibody-like protein of the present invention, the protein may be formed by self-assembly of 24 CDR-ferritin monomers.

In the antibody-like protein of the present invention, the protein may have a spherical shape with a particle diameter of 8 to 50 nm.

In the antibody-like protein of the present invention, the affinity (Kd) to the antigen bound to the complementarity determining region may be 1000 nM or less.

In the antibody-like protein of the present invention, the antibody may be an antibody against any one selected from the group consisting of PD-1, Her-2/neu, VISTA, 4-1BBL, CD48, Galectin-9, Adenosine A2a receptor, CD80, CD86, ICOS, ICOSL, BTLA, OX-40L, CD155, BCL2, MYC, PP2A, BRD1, BRD2, BRD3, BRD4, BRDT, CBP, E2F1, MDM2, MDMX, PPP2CA, PPM1D, STAT3, IDH1, PD-L1, PD-L2, CD40L, LAG3, TIM3, TIGIT, BTLA, CD52, SLAMF7, 4-1BB, OX-40, ICOS, GITR, CD27, CD28, CD16, CD3, CD20, EGFR family, AXL, CSF1R, DDR1, DDR2, EPH receptor family, FGFR family, VEGFR family, IGF1R, LTK, PDGFR family, RET, KIT, KRAS, NTRK1, NTRK2 and SARS-Cov.

In the antibody-like protein of the present invention, the antibody may be an antibody against any one selected from the group consisting of gp100, MART-1, Melna-A, MAGE-A3, MAGE-C2, Mammaglobin-A, proteinsase-3, mucin-1, HPV E6, LMP2, PSMA, GD2, hTERT, PAP, ERG, NA17, ALK, GM3, EPhA2, NA17-A, TRP-1, TRP-2, NY-ESO-1, CEA, CA 125, AFP, Survivin, AH1, ras, G17DT, MUC1, Her-2/neu, E75, p53, PSA, HCG, PRAME, WT1, URLC10, VEGFR1, VEGFR2, E7, Tyrosinase peptide, B16F10, EL4 and neoantigen.

In the antibody-like protein of the present invention, the antibody may be an antibody against an antigen of a cancer selected from the group consisting of brain cancer, head and neck cancer, bladder cancer, breast cancer, cervical cancer, colon cancer, colorectal cancer, endometrial cancer, esophageal cancer, leukemia, lung cancer, liver cancer, ovarian cancer, pancreatic cancer, prostate cancer, rectal cancer, kidney cancer, gastric cancer, testicular cancer, uterine cancer, vascular tumor, squamous cell carcinoma, adenocarcinoma, small cell carcinoma, melanoma, glioma, neuroblastoma, sarcoma, laryngeal cancer, parotid cancer, biliary tract cancer, thyroid cancer, actinic keratosis, acute lymphocytic leukemia, acute myeloid leukemia, adenoid cystic carcinoma, adenoma, adenoid squamous cell carcinoma, anal canal cancer, anal cancer, anorectal cancer, astrocytoma, ganglion adenocarcinoma, basal cell carcinoma, bile cancer, bone cancer, bone marrow cancer, bronchial cancer, bronchial carcinoma, carcinoid, cholangiocarcinoma, chronic lymphocytic leukemia, chronic myelogenous leukemia, clear cell carcinoma, connective tissue cancer, cystic adenoma, digestive system cancer, duodenal cancer, endocrine system cancer, endoderm sinus tumor, endometrial hyperplasia, endometrial adenocarcinoma, endothelial cell carcinoma, ependymal cell, epithelial cell carcinoma, orbital cancer, focal nodular hyperplasia, gallbladder cancer, palpebral cancer, gastrobasal cancer, gastrinoma, glioblastoma, glucagonoma, heart cancer, hemangioblastoma, hemangioendothelioma, hemangioma, hepatodenoma, liver adenoma, hepatobiliary cancer, hepatocellular carcinoma, Hodgkin's disease, ileal cancer, insulinoma, intraepithelial neoplasia, intraepithelial squamous cell neoplasia, intrahepatic biliary tract cancer, invasive squamous cell carcinoma, jejunum cancer, joint cancer, pelvic cancer, giant cell carcinoma, colon cancer, lymphoma, malignant mesothelial cell tumor, medulloblastoma, medullary epithelioma, meningeal cancer, mesothelial cancer, metastatic carcinoma, oral cancer, mucoepithelial carcinoma, multiple myeloma, muscle cancer, nasal duct cancer, nervous system cancer, non-epithelial skin cancer, non-Hodgkin's lymphoma, soft cell carcinoma, oligodendroglioma, oral cancer, osteosarcoma, papillary serous adenocarcinoma, penile cancer, pharyngeal cancer, pituitary tumor, plasmacytoma, pseudosarcoma, pulmonary blastoma, rectal cancer, renal cell carcinoma, respiratory system cancer, retina blastoma, serous carcinoma, sinus cancer, skin cancer, small cell carcinoma, small intestine cancer, smooth muscle cancer, soft tissue cancer, somatostatin-secreting tumor, spine cancer, squamous cell carcinoma, striatal muscle cancer, submesothelial carcinoma, T cell leukemia, tongue cancer, ureter cancer, urethral cancer, cervical cancer, uterine trunk cancer, vaginal cancer, VIPoma, vulvar cancer, highly differentiated carcinoma, and Wilm's tumor.

In the antibody-like protein of the present invention, the antibody may be an antibody against an infectious disease antigen.

In the antibody-like protein of the present invention, the infectious disease may be a bacterial, fungal, viral, parasitic or prion-induced disease.

According to another aspect of the present invention, there is provided a pharmaceutical composition for treatment or prevention of a disease, including the above antibody-like protein.

In addition, according to another aspect of the present invention, there is provided a composition for diagnosis of a disease, including the above antibody-like protein.

Further, according to another aspect of the present invention, there is provided a method for detecting an antigen, including treating an antigen with the above antibody-like protein.

[Advantageous effects]

The antibody-like protein of the present invention has a structure completely different from that of an antibody or fragment (scFv) thereof.

The antibody-like protein of the present invention has a high density of complementarity determining regions on its surface or outside, such that only a portion of the complementarity determining region can bind to an antigen with a similar level of affinity (binding force) to an antibody.

The antibody-like protein of the present invention may be designed so that the complementarity determining region is disposed on the surface or outside of the ferritin protein in consideration of a distance between the complementarity determining regions of the antibody.

The antibody-like protein of the present invention may perform a role similar to that of multiple antibodies, such as a dual antibody.

The antibody-like protein of the present invention has advantageous structural properties for simultaneous binding with a plurality of antigens, and thus has significantly improved avidity (binding activity) compared to the antibody.

The antibody-like protein of the present invention includes only CDR of an antibody or a conservative sequence variant thereof, and does not induce an unnecessary immune response due to use of the entire antibody sequence other than the CDR.

The antibody-like protein of the present invention may be easily produced in a microorganism.

The antibody-like protein of the present invention is easy to store.

[Brief Description of Drawings]

FIG. 1 shows positions and density of the complementarity determining region displayed on the surface of an antibody-like protein of the present invention when the complementarity determining region is fused to the corresponding site of a CDR-ferritin monomer.

FIG. 2A shows a distance between the complementarity determining regions of a Tecentriq antibody.

FIG. 2B shows a distance between the respective CDRs within a symmetric structure formed when the complementarity determining region is fused to the corresponding site of the CDR-ferritin monomer.

FIG. 3 is a schematic diagram of a vector in which antibody CDR3s are fused to various positions of the ferritin monomer.

FIG. 4 is a schematic diagram of a vector for production of a heavy chain CDR-ferritin monomer (huHF-αPD-L1 HCDR3) fused with αPD-L1 HCDR3, and confirmation of the preparation and self-assembly of the monomer.

FIG. 5 is a schematic diagram of a vector for production of a heavy chain CDR-ferritin monomer (huHF-αPD1 HCDR3) fused with αPD1 HCDR3, and confirmation of the preparation and self-assembly of the monomer.

FIG. 6 is a schematic diagram of a vector for production of a heavy chain CDR-ferritin monomer (huHF-αCTLA4 HCDR3) fused with αCTLA4 HCDR3, and confirmation of the preparation and self-assembly of the monomer.

FIG. 7 is a schematic diagram of a vector for production of a heavy chain CDR-ferritin monomer (huHF-αTIGIT HCDR3) fused with αTIGIT HCDR3, and confirmation of the preparation and self-assembly of the monomer.

FIG. 8 is a schematic diagram of a vector for production of a heavy chain CDR-ferritin monomer (huHF-αLAG3 HCDR3) fused with αLAG3 HCDR3, and confirmation of the preparation and self-assembly of the monomer.

FIG. 9 is a schematic diagram of a vector for production of a heavy chain CDR-ferritin monomer (huHF-αTIM3 HCDR3) fused with αTIM3 HCDR3, and confirmation of the preparation and self-assembly of the monomer.

FIG. 10 is a schematic diagram of a vector for the preparation of a heavy chain CDR-ferritin monomer (huHF-αPD-L1-αTIGIT) in which αPD-L1 HCDR3 and αTIGIT HCDR3 are fused, and confirmation of the preparation and self-assembly of the monomer.

FIG. 11 is a schematic diagram of a vector in which various antibody CDR3s are fused to the C-terminus of the ferritin monomer.

FIG. 12 is a schematic diagram of a vector for production of a heavy chain CDR-ferritin monomer (huHF-PD1 (Tecentriq) HCDR3) fused with PD1 (Tecentriq) HCDR3, and confirmation of the preparation and self-assembly of the monomer.

FIG. 13 is a schematic diagram of a vector for production of a heavy chain CDR-ferritin monomer (huHF-PD1 (keytruda) HCDR3) fused with PD1 (keytruda) HCDR3, and confirmation of the preparation and self-assembly of the monomer.

FIGS. 14 to 35 are graphs showing plots that measure the antigen-affinity (A) of the prepared antibody-like proteins.

FIG. 36 shows a schedule for assessment of tumor suppressive ability of the protein.

FIGS. 37 and 38 show results of evaluating tumor inhibitory ability of a heavy chain CDR-ferritin monomer (huHF-PD-L1-TIGIT dual blocker) fused with αPD-L1 HCDR3 and αTIGIT HCDR3.

FIGS. 39 and 40 show comparison results of tumor inhibitory ability according to the fusion position of α-PD-L1 HCDR3 to the ferritin monomer.

FIGS. 41 and 42 show results of evaluating the antigen-affinity of the antibody-like protein in cells.

[Mode for Carrying out Invention]

In one embodiment of the present invention, there is provided an antibody-like protein, in which a complementarity determining region of an antibody is formed by self-assembly of a plurality of ferritin monomers including at least a fused CDR-ferritin monomer, such that the complementarity determining region is present at a high density on the surface or outside of HCDR3, etc., wherein the antibody-like protein has significantly improved avidity compared to antibodies since it can bind to an antigen with a similar level of affinity to an antibody with only the complementarity determining region of the antibody and has advantageous structural properties for simultaneous binding with a plurality of antigens.

The antibody-like protein of the present invention is formed by self-assembly of ferritin monomers. In eukaryotic ferritin, 24 monomers come together to form a spherical three-dimensional structure.

The ferritin monomer useable in the present invention is not limited in origin and sequence. The ferritin monomer of the present invention includes human ferritin monomers. Human heavy chain ferritin monomer or human light chain ferritin monomer may be used as the human ferritin monomer. The human heavy chain ferritin monomer and human light chain ferritin monomer may also be used together.

Human ferritin consists of a heavy chain (21 kDa) and a light chain (19 kDa), and 24 monomers form a self-assembly.

The human ferritin has an outer diameter of about 12 nm and an inner diameter of about 8 nm. A structure of the ferritin monomer may include a form in which five α-helix structures, that is, A helix, B helix, C helix, D helix and E helix, are sequentially connected, and a non-canonical polypeptide moiety to link polypeptides each having α-helix structure called a loop is included.

The loop refers to a region that is not structurally broken even when the complementarity determining region is inserted into the ferritin monomer. In the present invention, a loop for linking A helix and B helix is called A-B loop, a loop for linking B helix and C helix is called B-C loop, a loop for linking C helix and D helix is called C-D loop, and a loop for linking D helix and E helix is called D-E loop.

Information on ferritin monomers is known from the NCBI (GenBank Accession No. NM_000146, NM_002032, etc.).

The ferritin monomer may be a ferritin heavy chain monomer, and specifically, a human ferritin heavy chain monomer. The human ferritin heavy chain monomer (huHF) may be a protein represented by the amino acid sequence of SEQ ID NO: 1 derived from human.

The ferritin heavy chain monomer may have some sequences mutated so that the affinity to the transferrin receptor is reduced. For example, in the case of a human ferritin heavy chain monomer, amino acids 15, 16, 23, 82 or 84 in the sequence of SEQ ID NO: 1 may be substituted with alanine, glycine, valine or leucine.

CDR-ferritin monomer is a ferritin monomer to which a complementarity determining region is fused. The antibody-like protein of the present invention may be formed by self-assembly of a plurality of ferritin monomers, wherein at least one CDR-ferritin monomer is included in the plurality of ferritin monomers. In this case, 24 or less of CDR-ferritin monomers may be included.

The complementarity determining region refers to any one selected from the group consisting of HCDR1, HCDR2, HCDR3, LCDR1, LCDR2 and LCDR3 of an antibody. The complementarity determining region may also include conservative sequence variants thereof.

The conservative sequence variant refers to variants of the amino acid sequence that do not significantly affect or change the binding properties of the complementarity determining region having a specific amino acid sequence, or have improved binding properties. Such conservative sequence variants may include substitutions, additions, and deletions of amino acids. Conservative amino acid substitutions may include substituting an amino acid residue with another amino acid residue having a similar side chain. A group of amino acid residues having similar side chains has been defined in the art. These groups may include amino acids having basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine).

The complementarity determining region is exposed to the surface on or outside of the antibody-like protein for binding with the antigen.

The complementarity determining region has a length capable of being fused to the ferritin monomer. For example, the length may be 25aa or less. If the length is 25aa or less, it is suitable for the complementarity determining region to be exposed to the surface or the outside after an antibody-like protein is formed by self-assembly without interfering with self-assembly by the ferritin monomer.

The length of the complementarity determining region may be, for example, 25aa or less, 20aa or less, 19aa or less, 18aa or less, 17aa or less, 16aa or less, 15aa or less, 14aa or less, 13aa or less, 12aa or less, 11aa or less, 10aa or less, 9aa or less, etc. Further, for example, it may be 3aa or more, 4aa or more, 5aa or more, 6aa or more.

The complementarity determining region is fused to any one selected from the group consisting of the inside of α-helix, between adjacent α-helices, N-terminus, C-terminus, A-B loop, B-C loop, C-D loop, D-E loop, between N-terminus and A helix, and between E helix and C-terminus. The position at which the complementarity determining region is to be fused may be determined among the above-listed sites of the ferritin monomer, in consideration of the three-dimensional arrangement of the complementarity determining region in the antibody, the distance therebetween, the degree of influence on the specific binding with the antigen and the like.

A CDR-ferritin monomer is a fusion of one or more complementarity determining regions. The CDR-ferritin monomer may be a fusion of at least one heavy chain complementarity determining region selected from the group consisting of HCDR1, HCDR2, HCDR3 and conservative sequence variants thereof of an antibody. Alternatively, the CDR-ferritin monomer may be a fusion of at least one light chain complementarity determining region selected from the group consisting of LCDR1, LCDR2, LCDR3, and conservative sequence variants thereof of an antibody.

The complementarity determining region for one antibody may be fused to one CDR-ferritin monomer or to a plurality of CDR-ferritin monomers.

One CDR-ferritin monomer may be fused with a complementarity determining region for one antibody, or complementarity determining regions for a plurality of antibodies.

From 1 to 24 CDR-ferritin monomers may participate in self-assembly. Alternatively, from 0 to 23 ferritin monomers to which the complementarity determining region is not fused may participate in self-assembly.

For example, 24 CDR-ferritin monomers may be self-assembled to form an antibody-like protein. For example, 12 CDR-ferritin monomers and 12 ferritin monomers to which complementarity determining regions are not fused may be self-assembled to form an antibody-like protein. For example, 12 first CDR-ferritin monomers for a first antibody and 12 second CDR-ferritin monomers for a second antibody may be self-assembled to form an antibody-like protein. For example, 12 heavy chain CDR-ferritin monomers to which heavy chain complementarity determining regions (HCDR1, HCDR2, HCDR3 or a conservative sequence variant thereof) for any antibody are fused, and 12 light chain CDR-ferritin monomers to which light chain complementarity determining regions (LCDR1, LCDR2, LCDR3 or a conservative sequence variant thereof) for the same antibody are fused, respectively, may be self-assembled to form an antibody-like protein. In addition, for example, 12 first CDR-ferritin monomers to which a plurality of complementarity determining regions of a first antibody group including a plurality of different antibodies are fused, and 12 second CDR-ferritin monomers to which a plurality of complementarity determining regions of a second antibody group including a plurality of different antibodies are fused, respectively, may be self-assembled to form an antibody-like protein.

Which position of the ferritin monomer the entire portion or a portion of the complementarity determining region is fused may be determined in consideration of the distance between the complementarity determining regions of the antibody, affinity and avidity to be required, and the contribution of each complementarity determining region to binding.

Depending on the number of self-assembled CDR-ferritin monomers, the number of complementarity determining regions included in the monomer, the fusion position of the complementarity determining regions, and the density of the complementarity determining regions after self-assembly, and the like, the affinity and avidity of an antibody-like protein may be adjusted.

Among the complementarity determining regions of the antibody, only a portion having major effects on the binding with an antigen may be selected and fused to a ferritin monomer. Depending on the type of antibody, if 1, 2, 3, 4 or 5 of total 6 complementarity determining regions have major effects on the binding with the antigen, only some of the complementarity determining regions having major effects may be fused to the ferritin monomer thus to prepare a CDR-ferritin monomer. For example, a CDR-ferritin monomer may be prepared by fusing only HCDR3 of an antibody to a ferritin monomer. For example, only HCDR3 and LCDR3 of an antibody may be selected and fused to a ferritin monomer thus to prepare a CDR-ferritin monomer.

When the complementarity determining region is fused to the DE loop or C-terminus of the CDR-ferritin monomer, it may form a four-fold symmetric structure. By utilizing such a four-fold symmetric structure, the arrangement and distance between the complementarity determining regions on the surface of an antibody-like protein may be made similar to the arrangement and distance between the complementarity determining regions of an antibody. For example, when 12 heavy chain CDR-ferritin monomers in which a heavy chain complementarity determining region (at least one of HCDR1, HCDR2, HCDR3 and conservative sequence variants thereof) is fused at the C-terminus of each ferritin monomer, and 12 light chain CDR-ferritin monomers in which a light chain complementarity determining region (at least one of LCDR1, LCDR2, LCDR3 and conservative sequence variants thereof) is fused at the C-terminus of each ferritin monomer, respectively, are self-assembled in the same ratio, the heavy chain complementarity determining region and the light chain complementarity determining region may be displayed on the surface of the antibody-like protein with being spaced apart from the antibody by a similar distance.

When the complementarity determining region is fused to the N-terminus, AB loop or BC loop of the CDR-ferritin monomer, a two-fold symmetric structure may be formed. By utilizing such a two-fold symmetric structure, the arrangement and distance between the complementarity determining regions on the surface of the antibody-like protein can be made similar to the arrangement and distance between the complementarity determining regions of an antibody. In particular, the BC loop is relatively long, such that it is suitable for fusing the complementarity determining regions at an appropriate position in consideration of the arrangement and distance between the complementarity determining regions. For example, when 12 heavy chain CDR-ferritin monomers in which HCDR1, HCDR2 and HCDR3 (or conservative sequence variants thereof) corresponding to the heavy chain complementarity determining regions are fused to the BC loop of each ferritin monomer while being spaced apart by a distance similar to that in an antibody, and light chain CDR-ferritin monomers in which LCDR1, LCDR2 and LCDR3 (or conservative sequence variants thereof) corresponding to the light chain complementarity determining regions are fused to the BC loop of another ferritin monomer with being spaced apart by a distance similar to that in the antibody, respectively, are self-assembled at the same ratio, the heavy chain complementarity determining region and the light chain complementarity determining region may be displayed on the surface of the antibody-like protein with being spaced apart therefrom by a distance similar to that of the antibody.

When the complementarity determining region is fused to the CD loop of the CDR-ferritin monomer, a three-fold symmetric structure may be formed. For example, when some complementarity determining regions (e.g., HCDR3) known to serve an important role in antigen binding are fused to the CD loop of a CDR-ferritin monomer, an antibody-like protein having a density of HCDR3 three (3) times higher than that of the antibody may be prepared.

Depending on which loop, terminus or helix of the ferritin monomer the complementarity determining region is fused to, and which position the loop, terminus or helix is fused to, a distance to the adjacent complementarity determining region may vary when the CDR-ferritin monomer is fused to form an antibody-like protein.

For example, the distance between the complementarity determining regions may be 0.7 nm, 0.75 nm, 0.8 nm, 0.85 nm, 0.9 nm, 0.95 nm, 1.0 nm, 1.05 nm. 1.1 nm, 1.15 nm, 1.2 nm, 1.25 nm, 1.3 nm, 1.35 nm, 1.4 nm, 1.45 nm, 1.5 nm, 1.55 nm, 1.6 nm, 1.65 nm, 1.7 nm, 1.75 nm, 1.8 nm, 1.85 nm, 1.9 nm, 1.95 nm, 2.0 nm, 2.05 nm, 2.1 nm, 2.15 nm, 2.2 nm, 2.25 nm, 2.3 nm, 2.35 nm, 2.4 nm, 2.45 nm, 2.5 nm, 2.55 nm, 2.6 nm, 2.65 nm, 2.7 nm, 2.75 nm, 2.8 nm, 2.85 nm, 2.9 nm, 2.95 nm, 3.0 nm, 3.05 nm, 3.1 nm, 3.15 nm, 3.2 nm, 3.25 nm, 3.3 nm, 3.35 nm, 3.4 nm, 3.45 nm, 3.5 nm, 3.55 nm, 3.6 nm, 3.65 nm, 3.7 nm, 3.75 nm, 3.8 nm, 3.85 nm, 3.9 nm, 3.95 nm, 4.0 nm, 4.05 nm, 4.1 nm, 4.15 nm, 4.2 nm, 4.25 nm, 4.3 nm, 4.35 nm, 4.4 nm, 4.45 nm, 4.5 nm, 4.55 nm, 4.6 nm, 4.65 nm, 4.7 nm, 4.75 nm, 4.8 nm, 4.85 nm, 4.9 nm, 4.95 nm, 5.0 nm, 5.05 nm, 5.1 nm, 5.15 nm, 5.2 nm, 5.25 nm, 5.3 nm, 5.35 nm, 5.4 nm, 5.45 nm, 5.5 nm, 5.55 nm, 5.6 nm, 5.65 nm, 5.7 nm, 5.75 nm, 5.8 nm, 5.85 nm, 5.9 nm, 5.95 nm, 6.0 nm, 6.05 nm, 6.1 nm, 6.15 nm, 6.2 nm, 6.25 nm, 6.3 nm, 6.35 nm, 6.4 nm, 6.45 nm, 6.5 nm, 6.55 nm, 6.6 nm, 6.65 nm, 6.7 nm, 6.75 nm, 6.8 nm, 6.85 nm, 6.9 nm, 6.95 nm or 7.0 nm

The antibody is a protein having a complementarity determining region. For example, antibodies, antigen-binding their fragments, and the like belonging to classes IgA, IGD, IgE, IgG, IgM, IgY, IgW, etc. may be included.

Further, the antibody may include an antibody to a disease antigen, an antibody to an immune checkpoint molecule, an antibody to an antigen to be detected for the purpose of diagnosis, prediction, evaluation, etc., or an antibody to an antigen that is a treatment target for a disease and the like.

The disease antigen may be an antigen causing a disease, a surface or internal antigen of a disease cell and the like. The diseased cells may be pathogen cells, pathogen-infected cells and the like.

The disease refers to any disease that can be treated by an antibody. For example, infectious diseases, cancer, inflammatory diseases, and the like may be included.

The infectious disease may be, for example, any disease caused by viruses, bacteria, fungi, parasites or prions.

Inflammatory diseases may include, for example, atherosclerosis, arteriosclerosis, autoimmune disease, multiple sclerosis, systemic lupus erythematosus, polymyalgia rheumatica, gouty arthritis, degenerative arthritis, tendinitis, bursitis, psoriasis, cystic fibrosis, osteoarthritis, rheumatoid arthritis, inflammatory arthritis, Sjogren's syndrome, giant cell arteritis, progressive systemic sclerosis (scleroderma), ankylosing spondylitis, polymyositis, dermatomyositis, pemphigus psoriasis, diabetes (e.g., type I), myasthenia gravis, Hashimoto's thyroiditis, Graves' disease, Goodpasture's disease, mixed connective tissue disease, sclerosing cholangitis, inflammatory bowel disease, Crohn's disease, ulcerative colitis, pernicious anemia, inflammatory dermatosis, common interstitial pneumonia, asbestosis, silicosis, bronchiectasis, beryllosis, talcosis, pneumoconiosis, sarcoidosis, dissecting interstitial pneumonia, lymphocytic interstitial pneumonia, giant cell interstitial pneumonia, cellular interstitial pneumonia, exogenous allergic alveolitis, Wegener's granulomatosis and related forms of vasculitis (temporal arteritis and polyarteritis nodosa), inflammatory dermatitis, hepatitis, delayed-type hypersensitivity reactions (e.g. poison ivy), pneumonia, respiratory tract infections, adult respiratory distress syndrome, encephalitis, immediate hypersensitivity reactions, asthma, hay fever, allergies, acute anaphylaxis, rheumatic fever, glomerulonephritis, pyelonephritis, cellulitis, cystitis, chronic cholecystitis, ischemia (ischemic injury), reperfusion injury, allograft rejection, host versus graft rejection, appendicitis, arteritis, blepharitis, bronchiolitis, bronchitis, cervicitis, cholangitis, chorioamnionitis, conjunctivitis, laryngitis, dermatomyositis, endocarditis, endometritis, enteritis, enterocolitis, epicondylitis, epididymitis, fasciitis, fibrosis, gastritis, gastroenteritis, gingivitis, ileitis, iritis, laryngitis, myelitis, myocarditis, nephritis, umbilical corditis, oophoritis, orchitis, osteitis, otitis, pancreatitis, parotitis, pericarditis, pharyngitis, pleurisy, phlebitis, pneumonia, proctitis, prostatitis, rhinitis, salpingitis, sinusitis, stomatitis, synovitis, orchitis, tonsillitis, urethritis, cystitis, uveitis, vaginitis, vasculitis, vulvitis, vulvovaginitis, vasculitis, chronic bronchitis, osteomyelitis, optic neuritis, temporal arteritis, transverse myelitis, necrotizing fasciitis and necrotizing enterocolitis.

For example, the cancer may be a cancer selected from the group consisting of brain cancer, head and neck cancer, bladder cancer, breast cancer, cervical cancer, colon cancer, colorectal cancer, endometrial cancer, esophageal cancer, leukemia, lung cancer, liver cancer, ovarian cancer, pancreatic cancer, prostate cancer, rectal cancer, kidney cancer, gastric cancer, testicular cancer, uterine cancer, vascular tumor, squamous cell carcinoma, adenocarcinoma, small cell carcinoma, melanoma, glioma, neuroblastoma, sarcoma, laryngeal cancer, parotid cancer, biliary tract cancer, thyroid cancer, actinic keratosis, acute lymphocytic leukemia, acute myeloid leukemia, adenoid cystic carcinoma, adenoma, adenoid squamous cell carcinoma, anal canal cancer, anal cancer, anorectal cancer, astrocytoma, ganglion adenocarcinoma, basal cell carcinoma, bile cancer, bone cancer, bone marrow cancer, bronchial cancer, bronchial carcinoma, carcinoid, cholangiocarcinoma, chronic lymphocytic leukemia, chronic myelogenous leukemia, clear cell carcinoma, connective tissue cancer, cystic adenoma, digestive system cancer, duodenal cancer, endocrine system cancer, endoderm sinus tumor, endometrial hyperplasia, endometrial adenocarcinoma, endothelial cell carcinoma, ependymal cell, epithelial cell carcinoma, orbital cancer, focal nodular hyperplasia, gallbladder cancer, palpebral cancer, gastrobasal cancer, gastrinoma, glioblastoma, glucagonoma, heart cancer, hemangioblastoma, hemangioendothelioma, hemangioma, hepatodenoma, liver adenoma, hepatobiliary cancer, hepatocellular carcinoma, Hodgkin's disease, ileal cancer, insulinoma, intraepithelial neoplasia, intraepithelial squamous cell neoplasia, intrahepatic biliary tract cancer, invasive squamous cell carcinoma, jejunum cancer, joint cancer, pelvic cancer, giant cell carcinoma, colon cancer, lymphoma, malignant mesothelial cell tumor, medulloblastoma, medullary epithelioma, meningeal cancer, mesothelial cancer, metastatic carcinoma, oral cancer, mucoepithelial carcinoma, multiple myeloma, muscle cancer, nasal duct cancer, nervous system cancer, non-epithelial skin cancer, non-Hodgkin's lymphoma, soft cell carcinoma, oligodendroglioma, oral cancer, osteosarcoma, papillary serous adenocarcinoma, penile cancer, pharyngeal cancer, pituitary tumor, plasmacytoma, pseudosarcoma, pulmonary blastoma, rectal cancer, renal cell carcinoma, respiratory system cancer, retina blastoma, serous carcinoma, sinus cancer, skin cancer, small cell carcinoma, small intestine cancer, smooth muscle cancer, soft tissue cancer, somatostatin-secreting tumor, spine cancer, squamous cell carcinoma, striatal muscle cancer, submesothelial carcinoma, T cell leukemia, tongue cancer, ureter cancer, urethral cancer, cervical cancer, uterine trunk cancer, vaginal cancer, VIPoma, vulvar cancer, highly differentiated carcinoma, and Wilm's tumor

The inflammatory disease antigen may be, for example, an inflammatory cytokine. For example, growth hormone such as human growth hormone, N-methionyl human growth hormone and bovine growth hormone; parathyroid hormone; thyroxine; insulin; proinsulin; relaxin; prorelaxin; glycoprotein hormones such as follicle stimulating hormone (FSH), thyroid stimulating hormone (TSH) and luteinizing hormone (LH); hepatic growth factor; fibroblast growth factor; prolactin; placental lactogen; tumor necrosis factor such as tumor necrosis factor-alpha (TNF-$\alpha$) and tumor necrosis factor-beta (TNF-$\beta$); mullerian-inhibitory substances; mouse gonadotropin-associated peptide; inhibin; activin; vascular endothelial growth factor; integrin; human platelet production promoter (thrombopoietin, TPO); nerve growth factors such as NGF-alpha (NGF-$\alpha$); platelet-growth factor; placental growth factor; transforming growth factors (TGF) such as TGF-$\alpha$ and TGF-$\beta$; insulin-like growth factor-1 and -11; erythropoietin (EPO); osteoinductive factors; interferons such as interferon-alpha (IFN-$\alpha$), interferon-beta (IFN-$\beta$) and interferon-gamma (IFN-$\gamma$); colony stimulating factors (CSF) such as macrophage-CSF (M-CSF); granulocyte macrophage-CSF (GM-CSF); granulocyte-CSF (G-CSF); interleukins such as IL-1, IL-2, IL-3, IL-4, IL-5, IL-6, IL-7, IL-8, IL-9, IL-10, IL-11, IL-12, IL- 13, IL-15, IL-17, IL-18, IL-21, IL-22, IL-23, IL-33, etc.; and other polypeptide factors including LIF and kit ligand (KL).

Cancer antigens may include, for example, gp100, MART-1, Melna-A, MAGE-A3, MAGE-C2, Mammaglobin-A, proteinsase-3, mucin-1, HPV E6, LMP2, PSMA, GD2, hTERT, PAP, ERG , NA17, ALK, GM3, EPhA2, NA17-A, TRP-1, TRP-2, NY-ESO-1, CEA, CA 125, AFP, Survivin, AH1, ras, G17DT, MUC1, Her-2/neu, E75, p53, PSA, HCG, PRAME, WT1, URLC10, VEGFR1, VEGFR2, E7, tyrosinase peptide, B16F10, EL4 neoantigen, etc. The neoantigen refers to an immunogenic peptide induced and formed by somatic mutation in tumor cells. The neoantigen forms a complex with MHC I, then moves to the surface of tumor cells, and may be displayed as an antigenic epitope. T-cell receptor (TCR) recognizes this neoantigen-MHC I complex and thus may induce an immune response.

The immune checkpoint molecule may be, for example, PD-L1, PD1, CTLA4, LAG3, TIM3 or TIGIT.

The antibody may be, for example, an antibody against an immune checkpoint molecule on the surface of a cancer cell or T cell. For example, the antibody may be PD-1, Her-2/neu, VISTA, 4-1BBL, CD48, Galectin-9, Adenosine A2a receptor, CD80, CD86, ICOS, ICOSL, BTLA, OX-40L, CD155, BCL2, MYC, PP2A, BRD1, BRD2, BRD3, BRD4, BRDT, CBP, E2F1, MDM2, MDMX, PPP2CA, PPM1D, STAT3, IDH1, PD-L1, PD-L2, CD40L, LAG3, TIM3, TIGIT, BTLA, CD52, SLAMF7, 4-1BB, OX-40, ICOS, GITR, CD27, CD28, CD16, CD3, CD20, EGFR family, AXL, CSF1R, DDR1, DDR2, EPH receptor family, FGFR family, VEGFR family, IGF1R, LTK, PDGFR family, RET, KIT, KRAS, NTRK1 or NTRK2

The antigen to be detected may be, for example, a biomarker, and may include, for example, a biomarker for disease diagnosis, a biomarker for predicting disease occurrence, a biomarker for prognostic diagnosis of a specific drug, etc., but it is not limited thereto.

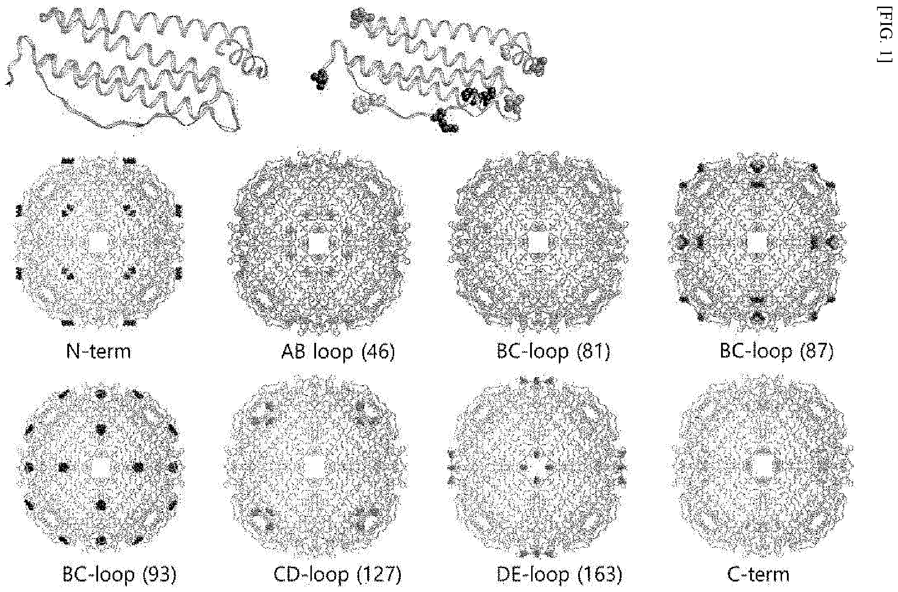

Hereinafter, the antibody-like protein of the present invention will be described with reference to FIG. 1.

FIG. 1 illustrates the structure of a protein consisting of self-assembly of 24 ferritin monomers, wherein ferritin has a plurality of symmetric structures including 4-fold, 3-fold and 2-fold symmetric structures. Herein, the N-terminus, AB loop and BC loop may form a 2-fold symmetric structure, the CD loop may form a 3-fold symmetric structure, and the DE loop and the C-terminus may form a 4-fold symmetric structure. The symmetric structure described herein refers to the number of flexible terminals gathered and, when the complementarity determining region is fused to the corresponding site, two, three or four complementarity determining regions may be gathered in the self-assembly. The parts indicated in each color in FIG. 1 show the fusion site when the complementarity determining region is fused to each site, and it could be seen that the arrangement, density, etc. of the protein to be fused by each site are different.

For example, according to self-assembly of 24 CDR-ferritin monomers fused with a complementarity determining region at the sites shown in FIG. 1, an antibody-like protein having four complementarity determining regions gathered at each of 6 sites may be obtained. Alternatively, by reducing the number of CDR-ferritin monomers, it is also possible to adjust the number of complementarity determining regions gathered in the above 6 sites. In addition, by regulating the fusion positions of the complementarity determining regions in a long loop (e.g., BC loop), a distance between the plurality of complementarity determining regions may be differently adjusted. This method may also be applied to a case even where a plurality of complementarity determining regions are fused to a CDR-ferritin monomer. Specifically, in order to more concentrate the plurality of homologous complementarity determining regions at a specific position when these are fused to a single antibody, otherwise, in order to mimic the structure of the complementarity determining region in an actual antibody when a heterologous complementarity determining region is fused to a single antibody, the complementarity determining region may be fused at a site having the 3-fold symmetric structure thus to control three (3) heterologous CDRs (e.g., HCDR1, HCDR2 and HCDR3, or LCDR1, LCDR2 and LCDR3) to gather in a short distance (e.g., fusion to CD loop).

The above regulation can be applied similarly even when a plurality of complementarity determining regions for a plurality of antibodies are fused. For example, when the complementarity determining regions of different antibodies are dense, antigen binding may be hindered by steric hindrance, and thus the complementarity determining regions of the different antibodies may be controlled not to be dense. This may be controlled, for example, by fusing the complementarity determining regions to different regions of each CDR-ferritin monomer in the first CDR-ferritin monomer and the second CDR-ferritin monomer to which the complementarity determining regions of different antibodies are fused respectively.

Further, for example, it is possible to regulate LCDR1, LCDR2 and LCDR3 to be dense, HCDR1, HCDR2 and HCDR3 to be gathered, or all of them to be gathered, such that the complementarity determining region of the same antibody mimics the structure of an antibody in the first CDR-ferritin monomer and the second CDR-ferritin monomer to which the complementarity determining regions are fused at the same site. The above process may be controlled by, for example, fusing the complementarity determining regions at the same site in the first and second CDR-ferritin monomers. For example, the above process may be controlled by fusing the CDRs at a site where a 3-fold symmetric structure is formed. This may be, for example, a C-D loop. For example, when HCDR1 of a specific antibody is fused to the C-D loop of the first CDR-ferritin monomer while HCDR2 and HCDR3 of the same antibody are fused to the C-D loop of the second CDR-ferritin monomer, and thus these are self-assembled, an antibody-like protein in which HCDR1, HCDR2 and HCDR3 are densed in the 3-fold symmetric structure, may be obtained.

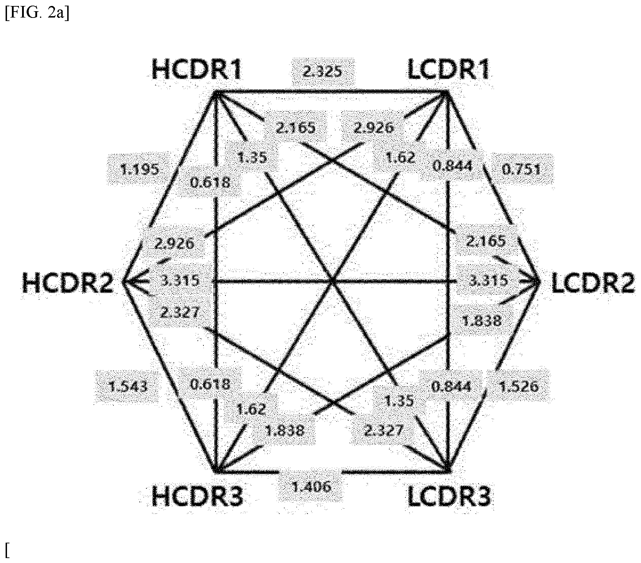

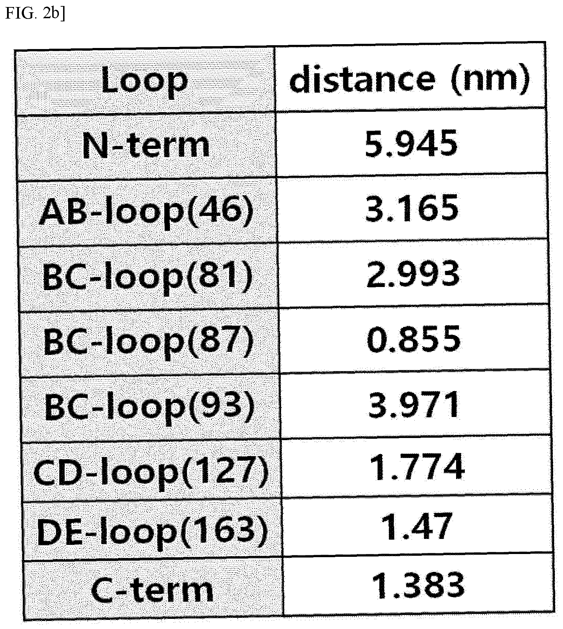

FIG. 2 shows the distance between CDRs of the commercially available antibody Tecentriq (atezolizumab) (upper part), and the distance between sites in the self-assembled protein of 24 human ferritin heavy chain monomers (lower part). This is a measurement of the distance between amino acid positions by designating the same on the 3D view of the protein data bank (www.rcsb.org). Specifically, the lower part of FIG. 2 shows the distances between the respective complementarity determining regions when the CDR-ferritin monomer is self-assembled, in the case where the complementarity determining regions are fused at N-terminus, AB loop; between 46D/47V, BC loop; between 81F/82L, between 87K/88K, 93D/94W, CD loop; between 127D/128P, DE loop; between 162E/163S, and at C-terminus, respectively. With reference to the above results, in order to measure the distance between the complementarity determining regions in a known antibody, and to mimic the distance between the complementarity determining regions, the density, etc., the CDRs of the CDR-ferritin monomer for the antibody may be fused at specific sites satisfying the distance in each antibody, thus to mimic the antibody.

The antibody-like protein of the present invention may exhibit excellent affinity to an antigen. The affinity can be variously adjusted depending on the fusion position of the complementarity determining regions, a degree of application of a plurality of complementarity determining regions, etc. For example, the affinity (Kd) to an antigen corresponding to the complementarity determining region of CDR-ferritin monomer may be 1000 nM or less. Within the above range, it may be 1000 nM or less, 900 nM or less, 800 nM or less, 700 nM or less, 600 nM or less, 500 nM or less, 400 nM or less, 300 nM or less, 200 nM or less, 150 nM or less, 100 nM or less, etc. The lower limit is not limited and may be, for example, 1 nM, 5 nM, 10 nM, 20 nM, 30 nM, 40 nM, 50 nM or the like. The affinity may be measured, for example, by an ELISA method.

When the antibody-like protein of the present invention forms particles, a particle diameter may be, for example, 8 to 50 nm. Specifically, it may be 8 to 50 nm, 8 to 45 nm, 8 to 40 nm, 8 to 35 nm, 8 to 30 nm, 8 to 25 nm, 8 to 20 nm, 8 to 15 nm, etc., but it is not limited thereto.

Since the antibody-like protein of the present invention uses only CDRs in the composition of an antibody as described above, it may not induce an unnecessary immune response due to use of the entire antibody sequence.

Further, since the ferritin protein can be stored at room temperature, the antibody-like protein of the present invention does not need to be refrigerated.

In addition, the present invention relates to a pharmaceutical composition for treating or preventing a disease, including the antibody-like protein.

It is natural to use the CDR of the antibody suitable for the target disease, the pharmaceutical composition for treatment of a disease according to the present invention is not only usable for the treatment of a specific disease, but also applicable to all diseases that can be alleviated, treated, or improved by the use of the antibody. As the disease to be treated herein, any disease known to be treatable by application of an antibody may be applied without limitation thereof, and may include, for example, an infectious disease, an inflammatory disease, cancer or the like. Specific examples thereof are the same as described above.

The CDR fused to the antibody-like protein of the present invention may be CDRs of an antibody to a disease antigen or an antibody to a therapeutic target substance in a disease.

The pharmaceutical composition of the present invention may further include an active substance known in the art for a target disease, a therapeutic agent and the like.

The active substances, therapeutic agents, etc., which can be additionally included, may be contained in the self-assembled structure, but they are not limited thereto.

The pharmaceutical composition of the present invention may include a pharmaceutically acceptable carrier. As used herein, the term "pharmaceutically acceptable carrier" refers to a carrier or diluent that does not significantly stimulate the organism and does not inhibit the biological activity and properties of the administered component. The pharmaceutically acceptable carrier in the present invention may include one component of saline, sterile water, Ringer's solution, buffered saline, dextrose solution, maltodextrin solution, glycerol, ethanol, and a mixture of one or more of these components. Other commonly used additives such as antioxidants, buffers and bacteriostatic agents may be added if necessary, thereby producing an injection formulation suitable for injection into tissues or organs. Further, it may be formulated as an isotonic sterile solution or, in some cases, a dry preparation (especially a freezedried preparation) that can become an injectable solution by adding sterile water or physiological saline to the administered component. In addition, a target organ-specific antibody or other ligands may be used in combination with the carrier so as to act specifically on the target organ.

Further, the composition of the present invention may further include a filler, excipient, disintegrant, binder or lubricant. Furthermore, the compositions of the present invention may be formulated using methods known in the art to provide rapid, sustained or delayed release of the active ingredient after administration to a mammal.

In one embodiment, the pharmaceutical composition may be an injection formulation and may be administered intravenously, but it is not limited thereto.

As used herein, the term "effective amount" refers to an amount necessary to delay the onset or progression of a specific disease to be treated or to completely improve the same.

In the present invention, the composition may be administered in a pharmaceutically effective amount. It is apparent to those skilled in the art that a proper total daily amount of the pharmaceutical composition can be determined by a physician for treatment within the scope of reasonable medical judgment.

For the purposes of the present invention, it is preferable that a specific pharmaceutically effective amount for a specific patient is differently applied depending on a specific composition, including the type and extent of the response to be achieved, in some cases, various factors including whether other agents are used, the specific composition, the patient's age, weight, general health status, gender and diet, administration time, administration route and secretion rate of the composition, treatment period, and drugs used or concurrently with the specific composition, as well as similar factors well known in the pharmaceutical field.

In the present invention, the pharmaceutical composition may be accompanied by instructions associated with packaging in a form instructed by a government agency in charge of the manufacture, use and sale of drugs if necessary, and the instructions indicate the approval of any private interest organization in relation of the form of a composition or administration to human or animals and may be, for example, a label approved by the US Food and Drug Administration for the prescription of a drug.

Further, the present invention relates to a composition for detecting a target substance, which includes the antibody-like protein.

The composition of the present invention includes the protein described above, and can confirm/detect the presence or absence of a target material based on whether to bind to the target material.

The target material may be, for example, a biomarker. For example, the target material may include a biomarker for diagnosing a disease, a biomarker for predicting the possibility of a disease, a biomarker for predicting the sensitivity of a specific drug, etc., but it is not limited thereto.

When the target substance is a biomarker for diagnosing a disease, the composition of the present invention may be used as a composition for diagnosing a disease.

The antibody CDR may be a CDR of an antibody to a target substance.

For easy detection/identification of the target substance, the antibody CDR, ferritin monomer or protein may be labeled with a fluorescent dye, a radioactive isotope, or the like, but it is not limited thereto.

The composition of the present invention may be treated with a sample to be checked for the presence or absence of a target substance, or treated with an animal.

The animal may be, for example, a mammal including a human, and specifically may be a human.

The composition of the present invention may be formulated in a variety of known methods, for example, may be formulated in the formulation exemplified above for the pharmaceutical composition, but it is not limited thereto.

The present invention also relates to a method for treating a disease, which includes administering an antibody-like protein to a subject.

The subject is a diseased individual, and may be a mammal including a human, and specifically may be a human.

The disease is a disease that can be treated by the use of an antibody, and may be the diseases exemplified above, but it is not limited thereto.

The antibody CDRs fused to the protein of the present invention may be CDRs of an antibody to a disease antigen or an antibody to a therapeutic target substance in a disease.

The protein may be administered in a therapeutically effective amount.

As used herein, the term "administration" means introducing the composition of the present invention to a patient by any suitable method, and the administration route of the composition of the present invention may include administration through various oral or parenteral routes as long as it can reach the target tissue. Specifically, it may include intraperitoneal administration, intravenous administration, intramuscular administration, subcutaneous administration, intradermal administration, oral administration, topical administration, intranasal administration, intrapulmonary administration or rectal administration, and the like, but it is not limited thereto.

Further, the present invention relates to a method for detecting an antigen, which includes treating the antibody-like protein.

The antibody CDRs may be CDR of an antibody to a target antigen.

For easy detection/identification of the target antigen, the antibody CDR, ferritin monomer, or protein may be labeled with a fluorescent dye, a radioisotope, or the like, but it is not limited thereto.

Hereinafter, the present invention will be described in more detail by way of the following examples in order to concretely describe the present invention.

## EXAMPLE

### 1. Construction of expression vector for protein production

(1) huHF is a spherical protein nanoparticle (12 nm) consisting of 24 monomers, wherein each monomer is composed of total 5 α-helixes. The present inventors ensured a delivery system, in which antibody CDR3 peptides were inserted at different positions in huHF, by inserting the antibody CDR3 peptide into a position selected among the loop between each α-helix of the huHF monomer (AB loop in huHF 5T to 176G based on the PDB 3AJO sequence; between 46D/47V, BC loop; 93D/94W, CD loop; between 127D/128P, DE loop; 163E/164S), N-terminus and C-terminus through gene cloning.

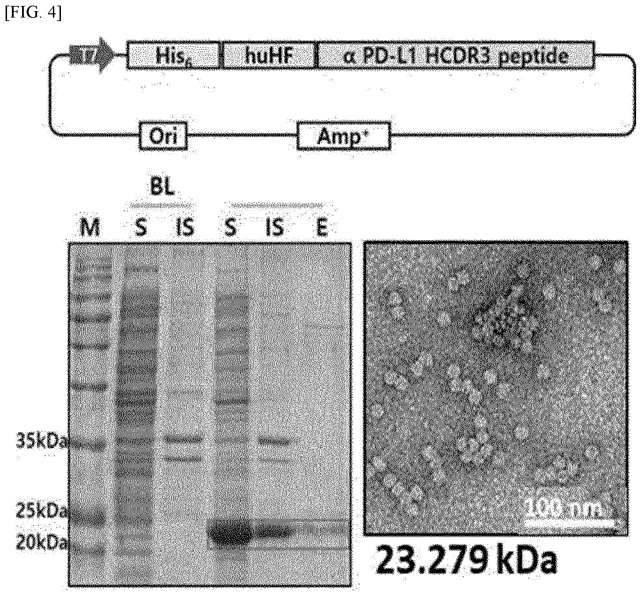

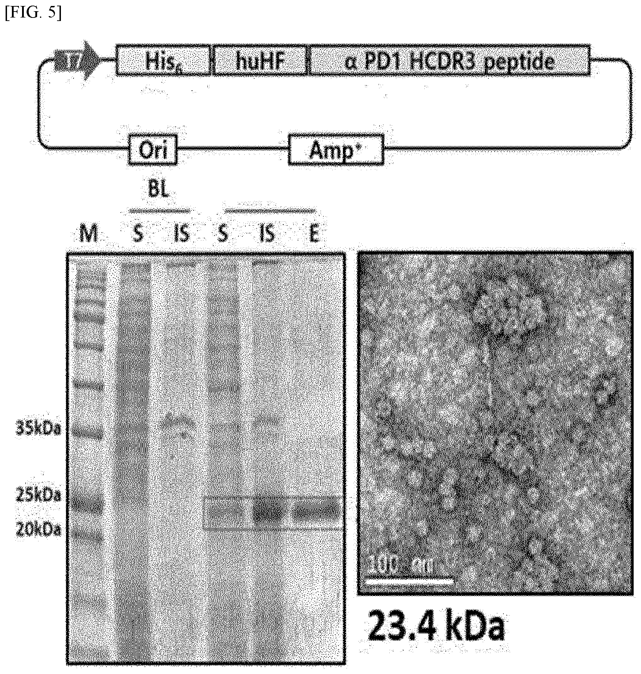

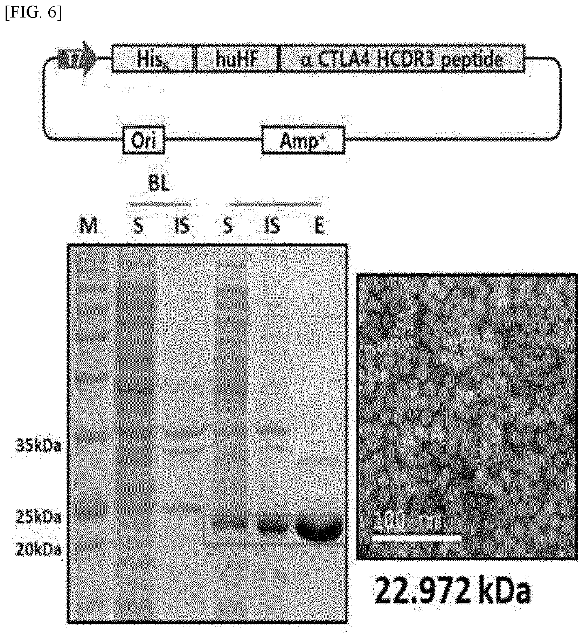

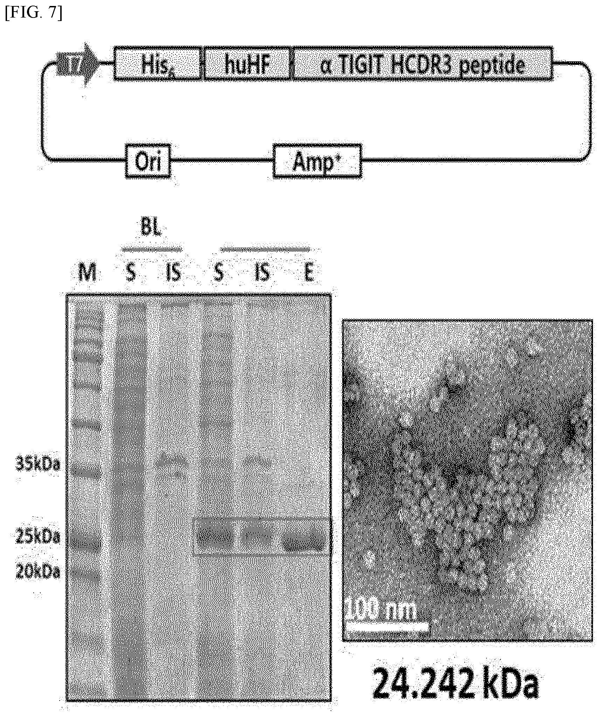

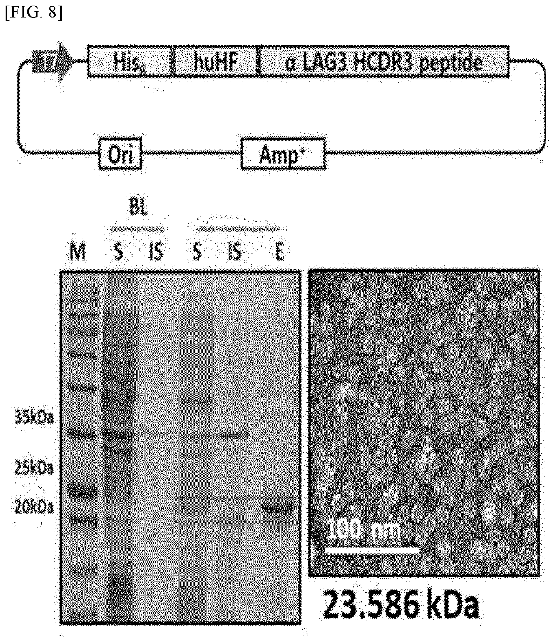

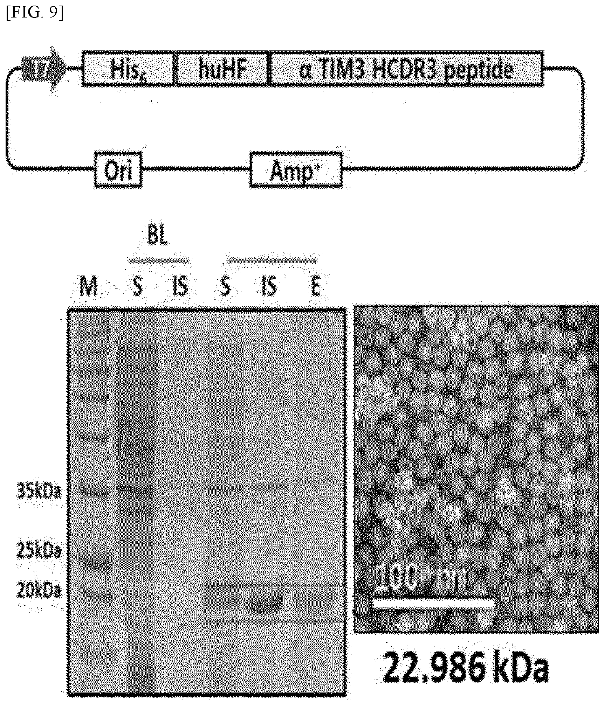

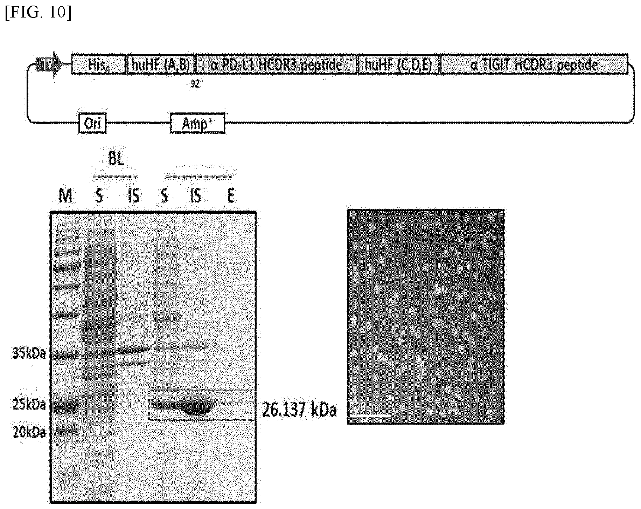

The sequences in Table 1 below were used, and PCR was executed according to a schematic diagram for vector preparation of FIGS. 4 to 10 and Table 2 below thus to prepare huHF-αPD-L1 HCDR3 (CD loops, C-terminus), huHF-αPD1 HCDR3 (C-terminus), huHF-αCTLA4 HCDR3 (C-terminus), huHF αTIGIT HCDR3 (C-terminus), huHF-αLAG3 HCDR3 (C-terminus), huHF-αTIM3 HCDR3 (C-terminus), huHF-αPD-L1 HCDR3 (AB loop)-αTIGIT HCDR3 (C-terminus) (dual blocker). All the constructed plasmid expression vectors were purified on an agarose gel, and then sequences were confirmed through complete DNA sequencing.

Specifically, an expression vector capable of expressing each protein nanoparticle was constructed by sequentially inserting the PCR products required for the preparation of each expression vector into the plasmid pT7-7 vector using the primer set of Table 3 below. In this case, the linker peptide of Table 3 below may be additionally included.

[TABLE 1]

| Sequence No. | Name | Sequence |
|---|---|---|
| 2 | αPD-L1 | GGSYGSLYAFDI |
| 3 | αPD1 | DLGAGPYYYGKDV |

(continued)

| Sequence No. | Name | Sequence |
|---|---|---|
| 4 | αCTLA4 | TAQFAY |
| 5 | αLAG3 | GYSDYEYNWFDP |
| 6 | αTIM3 | VGGAFPMDY |
| 7 | αTIGIT | MPSFITLASLSTWEGYFDF |

[TABLE 2]

| CDR-ferritin monomer | Expression vector |
|---|---|
| huHF-αPD-L1 | NH2-NdeI-huHF-αPD-L1 HCDR3-HindIII-COOH |
| huHF-αPD1 | NH2-NdeI-huHF-αPD1 HCDR3-HindIII-COOH |
| huHF-αCTLA4 | NH2-NdeI-huHF-αCTLA4 HCDR3-HindIII-COOH |
| huHF-αLAG3 | NH2-NdeI-huHF-αLAG3 HCDR3-HindIII-COOH |
| huHF-αTIM3 | NH2-NdeI-huHF-αTIM3 HCDR3-HindIII-COOH |
| huHF-αTIGIT | NH2-NdeI-huHF-αTIGIT HCDR3-HindIII-COOH |
| huHF-PD-L1-TIGIT dual blocker | BC(92D/93W): NH2-NdeI-(His)6-huHF-[αTIGIT HCDR3]-huHF- αPD-L1 HCDR3-HindIII-COOH |

[TABLE 3]

| Sequence No. | Name | Insertion site | Primer |
|---|---|---|---|
| 8 | α_PD-L1_F | C-terminus | CATATGCACCATCACCATCACCATACGACCGCGTCC |
| 9 | α_PD-L1_R | | AAGCTTCTAAATATCAAACGCATACAGAGAACCATAAGAACCACCGCTTTCATTATC |
| 10 | α_PD1_F | C-terminus | CATATGCACCATCACCATCACCATACGACCGCGTCC |
| 11 | α_PD1_R | | AAGCTTCTACACATCTTTGCCATAGTAATACGGGCCCGCGCCCAGATCGCTTTCATTATC |
| 12 | α_CTLA4_F | C-terminus | CATATGCACCATCACCATCACCATACGACCGCGTCC |
| 13 | α_CTLA4_R | | AAGCTTCTAATACGCAAACTGCGCGGTCTCGAGGCTTTCATTATC |
| 14 | α_LAG3_F | C-terminus | CATATGCACCATCACCATCACCATACGACCGCGTCC |
| 15 | α_LAG3-R | | AAGCTTCTACGGATCAAACCAGTTATATTCATAATCGCTATAGCCGCTTTCATTATC |
| 16 | α_TIM3_F | C-terminus | CATATGCACCATCACCATCACCATACGACCGCGTCC |
| 17 | α_TIM3_R | | AAGCTTCTAATAATCCATCGGAAATGCACCGCCAACGCTTTCATTATC |

(continued)

| Sequence No. | Name | Insertion site | Primer |
|---|---|---|---|
| 18 | α_TIGIT_F | C-terminus | CATATGCACCATCACCATCACCATACGACCGCGTCC |
| 19 | α_TIGIT_R | | AAGCTTCTAAAAATCAAAATAGCCTTCCCAGGTGCTCAGGCTCGCCAGGGTAATAAAGCTCGGCATGCTTTCATTATC |
| 20 | BC_α_PD-L1_F | BC loop | CTGTATGCGTTTGATATTGAATTCTGGGAGAGCGGGCTGAAT |
| 21 | BC_α_PD-L1_R | | AGAACCATAAGAACCACCGGATCCGTCATCACAGTCTGGTTTCTTGATATC |

[TABLE 4]

| Sequence No. | Name | Sequence |
|---|---|---|
| 22 | Linker1 | GGGSGGGT |
| 23 | Linker2 | GGGGSGGGGT |
| 24 | Linker3 | GGGSGGGTGGGS |

(2) An antibody CDR3 peptide was inserted into a position selected among the loop between each α-helix of huHF monomer (AB loop in huHF 5T to 176G based on PDB 3AJO sequence; 46D/47V, BC loop; 81F/82L, 87K/88K, 93D/94W, CD loop; Between 127D/128P, DE loop; between 162E/163 S), N-terminus and C-terminus through gene cloning, thereby ensuring a delivery system in which the antibody CDR3 peptides were inserted at different positions in huHF.

As huHF, mutant huHF of SEQ ID NO: 25 was used.

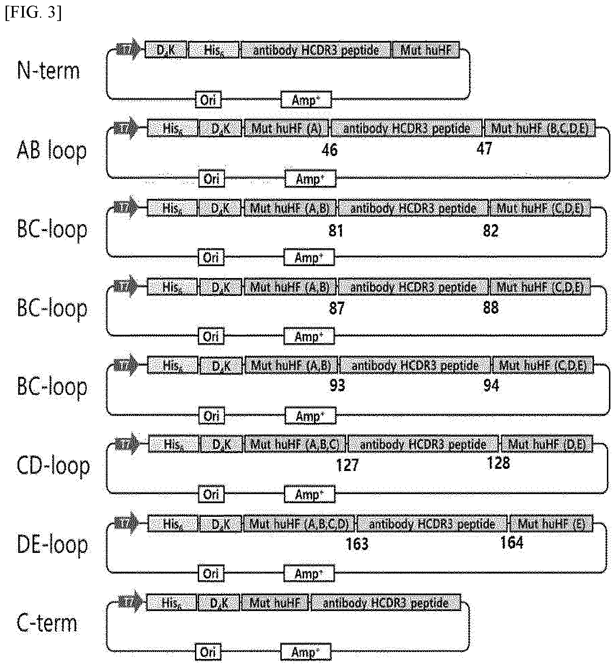

The sequences in Table 5 below were used as CDRs and, according to the schematic view for vector preparation shown in FIG. 3, huHF-Her2/neu (herceptin) HCDR3 (C-terminus), huHF-PD1 (keytruda) HCDR3 (C-terminus), huHF-PD-L1 (tecentriq) HCDR3 (C-terminus), huHF-PD-L1 HCDR3 (N-terminus, AB loop, BC loop (81/82), BC loop (87/88), BC loop (93/94), DE loop or (C-terminus), huHF-TIM3 HCDR3 (C-terminus), huHF-LAG3 HCDR3 (C-terminus), huHF-TIGIT HCDR3 (C-terminus), huHF-SARS-Cov HCDR3 (C-terminus), huHF-PD-L1 HCDR3 (AB loop)-αTIGIT HCDR3 (C-terminus) were prepared.

All of the constructed plasmid expression vectors were purified on an agarose gel, and then, the sequence was confirmed through complete DNA sequencing. As a plasmid vector, pT7-7 vector was used.

[TABLE 5]

| Sequence No. | Name | Sequence |
|---|---|---|
| 26 | Her2/neu (herceptin) | WGGDGFYAMDY |
| 27 | PD1 (keytruda) | RDYRFDMGFDY |
| 28 | PD-L1(tecentriq) | RHWPGGF |
| 2 | PD-L1 | GGSYGSLYAFDI |
| 5 | LAG3 | GYSDYEYNWFDP |
| 6 | TIM3 | VGGAFPMDY |
| 7 | TIGIT | MPSFITLASLSTWEGYFDF |
| 29 | SARS-Cov | ARGDSSGYYYYFDY |
| 30 | CTLA4(yervoy) | ARTGWLGPFDY |

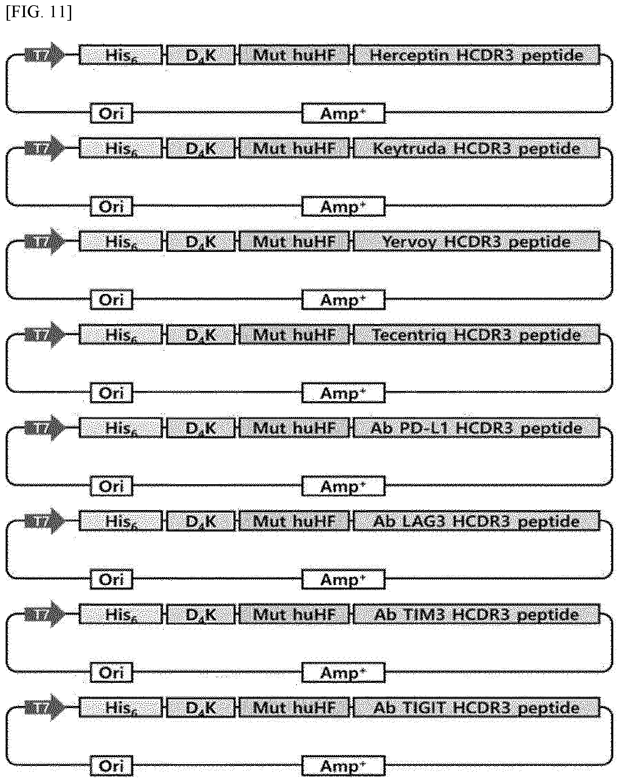

(3) According to the schematic view for vector preparation shown in FIG. 11, huHF-Her2/neu (herceptin) HCDR3 (C-terminus), huHF-PD1 (keytruda) HCDR3 (C-terminus), huHF-CTLA4 (yervoy) HCDR3 (C-terminus), huHF-PD-L1 (tecentriq) HCDR3 (C-terminus), huHF-PD-L1 HCDR3 (C-terminus), huHF-LAG3 HCDR3 (C-terminus), huHF-TIM3 HCDR3 (C-terminus), huHF-TIGIT HCDR3 were prepared.

All of the constructed plasmid expression vectors were purified on an agarose gel, and then the sequence was confirmed through complete DNA sequencing. As a plasmid vector, pT7-7 vector was used.

## 2. Protein biosynthesis

*E. coli* strain BL21(DE3)[F-ompThsdSB(rB-mB-)] was transformed with the expression vector prepared above, respectively, and ampicillin-resistant transformants were selected. The transformed *E. coli* was cultured in a flask (250 mL Erlenmeyer flasks, 37 °C, 150 rpm) containing 50 mL of Luria-Bertani (LB) medium (containing 100 mg L-1 ampicillin). When the medium turbidity (O.D 600) reached about 0.5-0.7, Isopropyl-β-Dthiogalactopyanosid (IPTG) (1.0 mM) was injected to induce expression of the recombinant gene.

Except for huHF-(93-AbPD-L1+C-AbTIGIT), individual expression vectors were used and transformed into one vector. In the case of huHF-(93-AbPD-L1+C-AbTIGIT), it was transformed into two vectors for the preparation of huHF-PD-L1 HCDR3 (BC loop (92/93)) and huHF-TIGIT HCDR3.

After incubation at 20 °C for 16 to 18 hours, the cultured *E. coli* was centrifuged at 4,500 rpm for 10 minutes to recover the cell precipitate, followed by suspending the product in 5 ml of a disruption solution (10 mM Tris-HCl buffer, pH 7.5, 10 mM EDTA) and crushing the same using an ultrasonic crusher (Branson Ultrasonics Corp., Danbury, CT, USA). After crushing, centrifugation was performed at 13,000 rpm for 10 minutes to separate the supernatant and insoluble aggregates. The separated supernatant was used for subsequent experiments.

## 3. Protein purification and fluorescent material attachment

The supernatant obtained in Example 2 above was purified through a three-step process. First, 1) $Ni^{2+}$-NTA affinity chromatography using the binding of histidine and nickel fused to the recombinant protein was performed, then 2) the recombinant protein was concentrated and the fluorescent material was attached through buffer exchange, and finally, 3) sucrose gradient ultracentrifugation was performed to separate only the fluorescent material-attached and self-assembled protein nanoparticles. The detailed description of each step is as follows.

1) $Ni^{2+}$-NTA affinity chromatography

In order to purify the recombinant protein, *E. coli* cultured in the same manner as specified above was recovered, the recovered cell pellets were resuspended in 5 mL lysis buffer (pH 8.0, 50 mM sodium phosphate, 300 mM NaCl, 20 mM imidazole), and the cells were crushed using a sonicator. The crushed and lysed cell solution was centrifuged at 13,000 rpm for 10 minutes to separate only the supernatant, and then each recombinant protein was separated using a $Ni^{2+}$-NTA column (Qiagen, Hilden, Germany) (washing buffer: pH 8.0, 50 mM sodium phosphate, 300 mM NaCl, 80 mM imidazole / elution buffer: pH 8.0, 50 mM sodium phosphate, 300 mM NaCl, 200 mM imidazole).

2) Concentration, buffer exchange, and fluorescent material attachment process

For imaging, huHF-gp100 particles and huHF-PD1 particles were subjected to $Ni^{2+}$- NTA affinity chromatography, and 3 ml of the eluted recombinant protein was placed in an ultracentrifugal filter (Amicon Ultra 100K, Millipore, Billerica, MA) at 5,000 g on the column. Centrifugation was performed at 5,000 g until 1 ml of the solution remained. Thereafter, the protein particles were buffer exchanged with sodium bicarbonate (0.1 M, pH 8.5) buffer in order to attach NIR fluorescent substances such as cy5.5 and fluorescein isothiocyanate (FITC), followed by attaching the fluorescent substance at room temperature for 12 hours.

3) Sucrose gradient ultracentrifugation

To PBS (2.7 mM KCl, 137 mM NaCl, 2 mM KH2PO4, 10 mM Na2HPO4, pH7.4) buffer, each concentration of sucrose was added to prepare solutions each containing 40%, 35%, 30%, 25%, and 20% sucrose, respectively. After preparing each solution, 2 ml of the solution at a concentration (45-20%), starting from the solution with the highest concentration, was put in a high-speed centrifugation tube (ultraclear 13.2 ml tube, Beckman), and then 1 ml of the recombinant protein solution existing in the prepared self-assembled buffer was added thereto, followed by ultra-high speed centrifugation at 35,000 rpm and 4° C for 16 hours (Ultracentrifuge L-90k, Beckman). After centrifugation, the upper layer (20-25% sucrose solution part) was subjected to replacement of the buffer in the recombinant protein by a pipette using an ultracentrifugal filter and PBS buffer as described in step 2).

### 4. Identification of water-soluble fractions of proteins

BL21 (DE3) competent cells were transformed with various pT7-7-based expression vectors. A single colony was inoculated in LB liquid medium (50 mL) supplemented with 100 mg/L of ampicillin and cultured under conditions of 37 °C and 130 rpm in a shaking incubator. When the turbidity/optical density at 600 nm reached 0.5, the expression of the target protein was induced through administration of 1 mM IPTG. After 12 to 16 hours of incubation at 20 °C, the cells in the culture medium were spun-down through centrifugation (13,000 rpm, 10 minutes), and the cell pellet was collected and resuspended in 10 mM Tris-Hcl (pH 7.4) buffer. The resuspended cells were ruptured using a Branson Sonifier (Branson Ultrasonics Corp., Danbury, CT). After sonication, the supernatant containing soluble protein and aggregates containing insoluble protein were separated by centrifugation (13,000 rpm, 10 min). Through SDS-PAGE analysis, the separated soluble and insoluble protein fractions were confirmed.

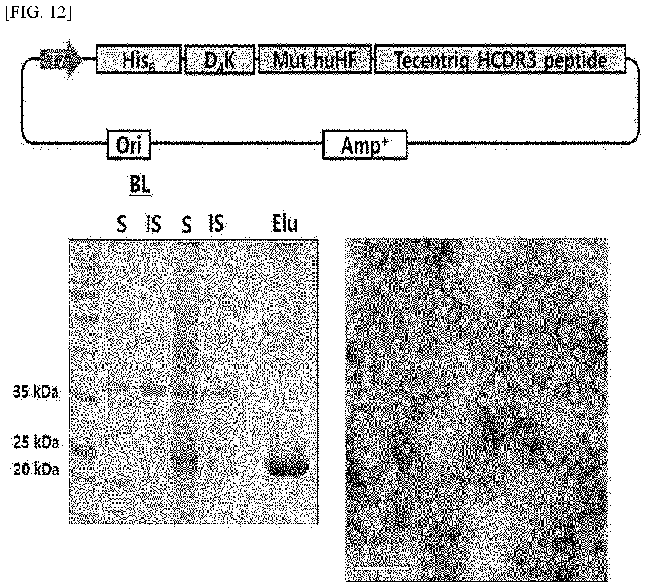

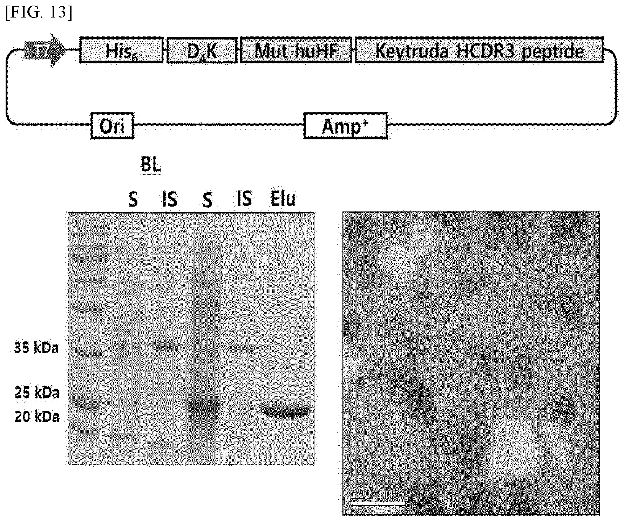

FIGS. 4 to 10 show products using the vector of Example 1.(1), and FIGS. 12 and 13 show products using the vector of Example 1.(3).

### 5. Verification of protein assembly

In order to analyze the structure of the purified recombinant protein nanoparticles of each protein prepared in Example 3, the recombinant protein was photographed with a transmission electron microscope (TEM). First, unstained and purified protein samples were placed on carbon-coated copper electron microscope grids and then air-dried. To obtain stained images of proteins, electron microscope grids including the air-dried samples were incubated with 2% (w/v) aqueous uranyl acetate solution for 10 minutes at room temperature and washed 3-4 times with distilled water. As a result of observing the protein image using a Philips Technai 120 kV electron microscope, it was confirmed that each particle forms a spherical nanoparticle (FIGS. 4 to 10, 12 and 13).

### 6. Measurement of protein affinity to antigen

The antigen-affinity of the proteins prepared in the above examples was compared with the antigen-affinity of the antibody. The affinity A of the prepared protein to the antigen was measured according to the following method.

Nunc MaxiSorpTM flat-bottom (cat. 44-2404-21) 96 well-plates were used.

100 $\mu$l of protein at a concentration of 2 $\mu$g/ml was dispensed into each well-plate, and the protein was attached by shaking the plate at 4° C over-night.

After removing the solution from the well-plate, 200 $\mu$l of PBST (Tween20, 0.05%) was dispensed and removed (washing).

200 $\mu$l of SuperBlock™ (PBS) Blocking Buffer (cat. 37515) was dispensed at a time for masking, and the solution was removed from the well-plate, followed by dispensing 200 $\mu$l of PBST (Tween20, 0.05%) and then removing the same (washing).

Thereafter, 100 $\mu$l of the sample to be checked for Kd was dispensed into each well-plate by the concentration and, after removing the solution from the well-plate, 200 $\mu$l of PBST (Tween20, 0.05%) was dispensed and removed (washing).

After 2nd antibody-HRP was diluted 1/100 in PBS, 100 $\mu$l of this solution was dispensed into each well-plate. After removing the solution from the well-plate, 200 $\mu$l of PBST (Tween20, 0.05%) was dispensed and removed (washing).

Thereafter, 100 $\mu$l of TMB solution was dispensed into each well-plate, and 100 $\mu$l of Stop solution (1M $H_2SO_4$) was dispensed into each well-plate, followed by measuring absorbance at 450 nm wavelength.

A graph of absorbance (abs) at each concentration was drawn to determine a fluorescence value (abs(sat)) at the saturation point, and further, a graph having concentration/abs on x-axis and concentration/abs(sat) on y-axis was drawn to prepare an equation through linearly fitting.

Multiplying y-intercept in the equation by abs(sat) may obtain Kd.

Each affinity indicates the affinity of the target antibody to the target antigen. In the figure, "m" in, for example, mPD-L1 indicates the affinity to mouse antigen, while "h" in, for example, hPD-L1 indicates the affinity to human antigen.

All of the antibody-like proteins in the examples were formed by self-assembly of 24 CDR-ferritin monomers. huHF-dual is a self-assembly of 24 heavy chain CDR-ferritin monomers fused with PD-L1 HCDR3 and TIGIT HCDR3, and huHF-(93-AbPD-L1+C-AbTIGIT) is a self-assembly including first heavy chain CDR-ferritin monomer fused with PD-L1 HCDR3 at position 93 (BC loop) and second heavy chain CDR-ferritin monomer fused with TIGIT HCDR3 at C-terminus, wherein a sum of the number of first and second heavy chain CDR-ferritin monomers is 24.

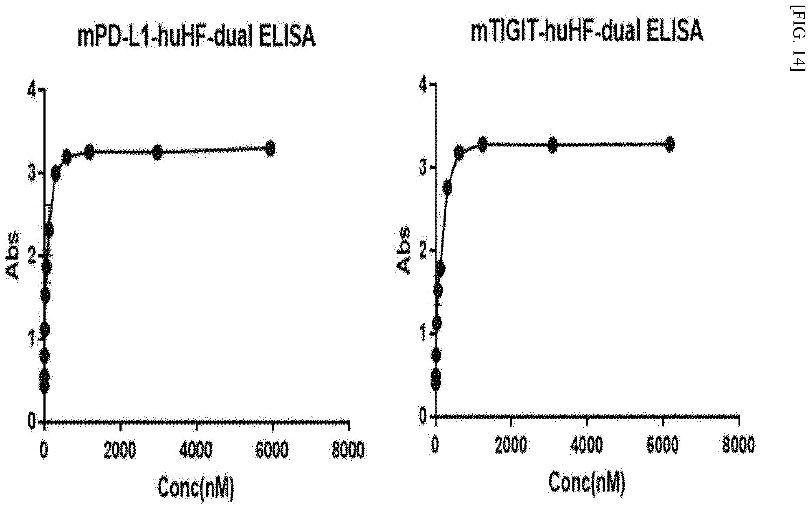

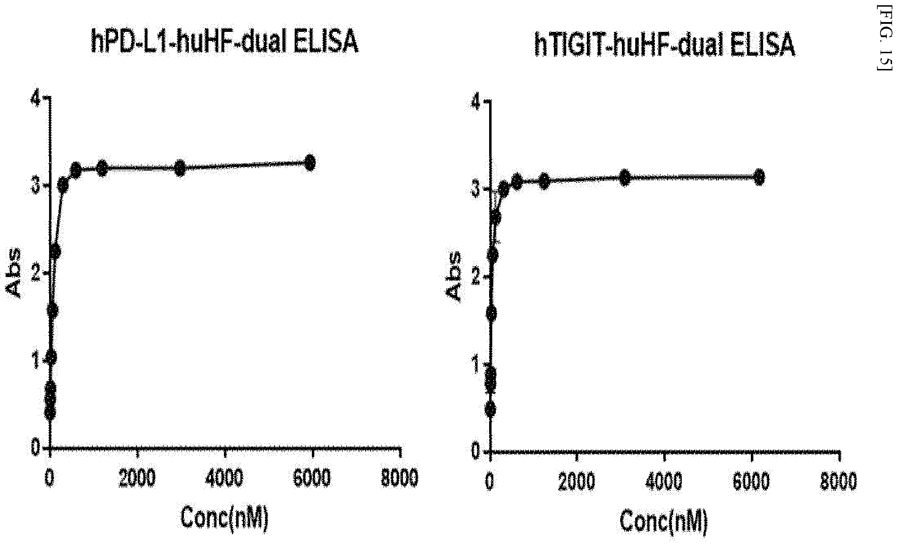

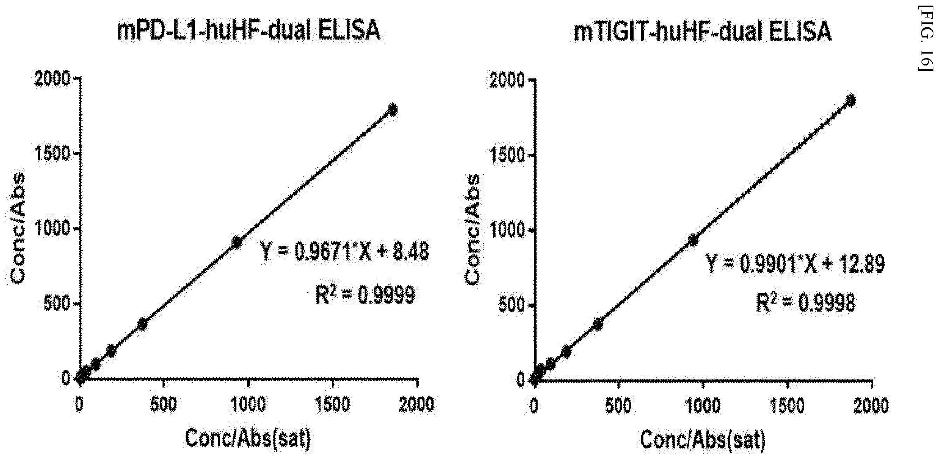

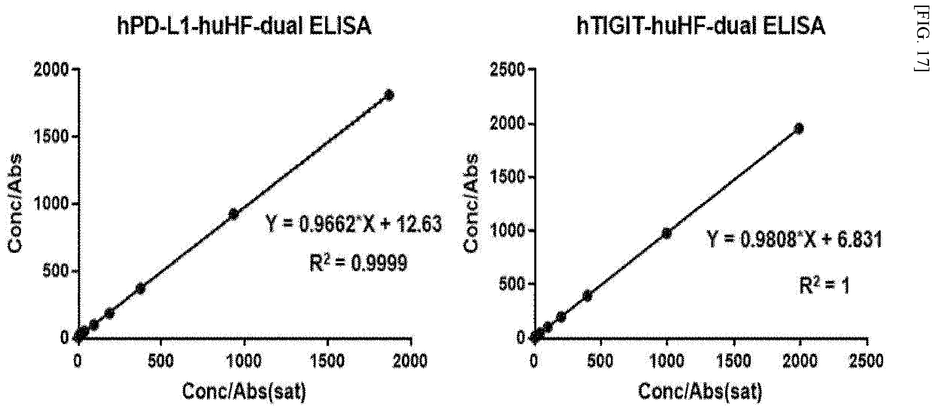

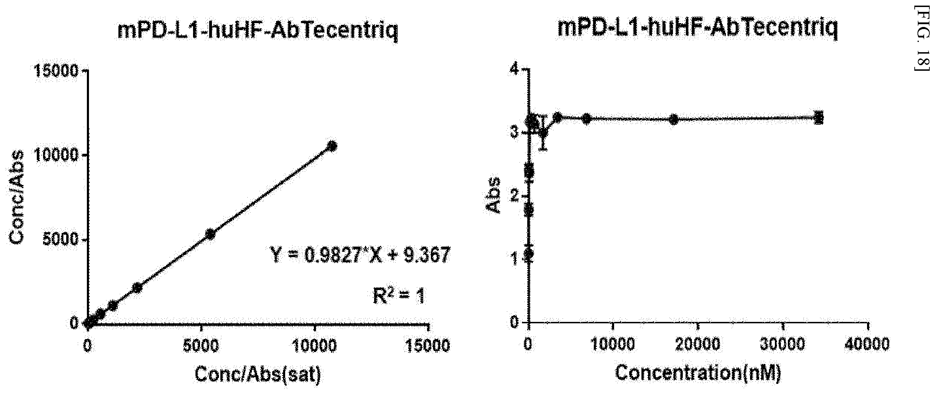

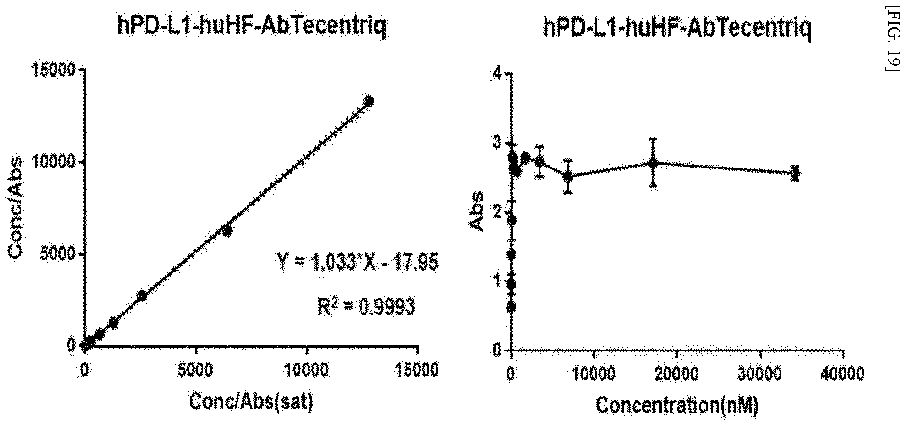

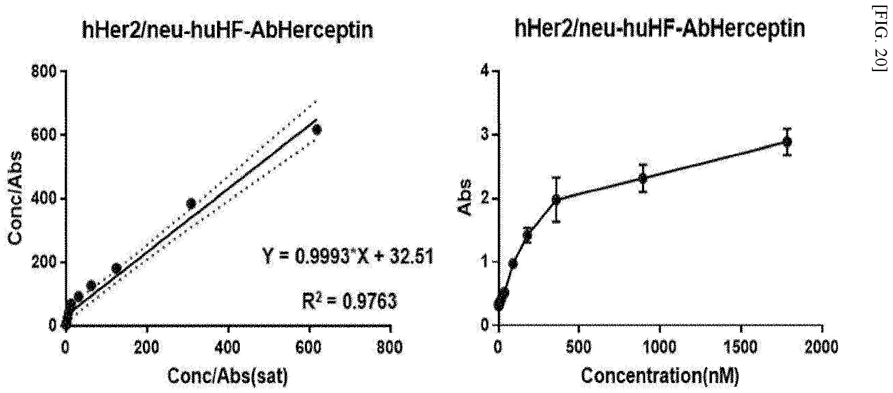

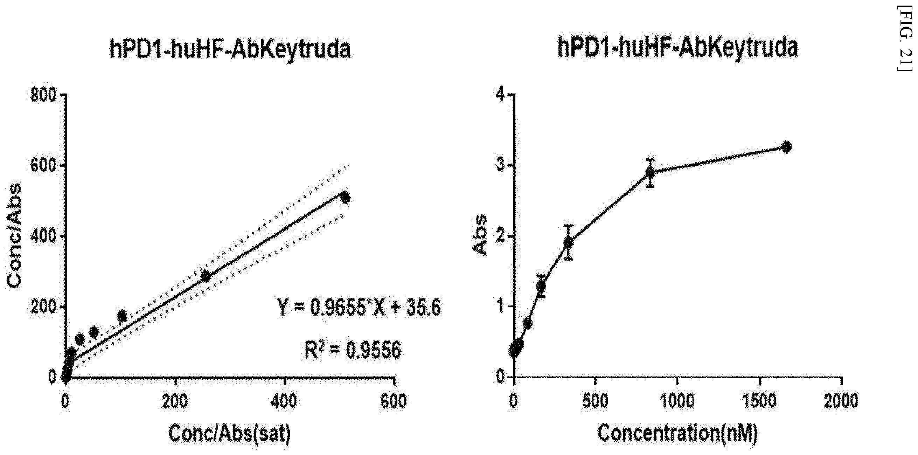

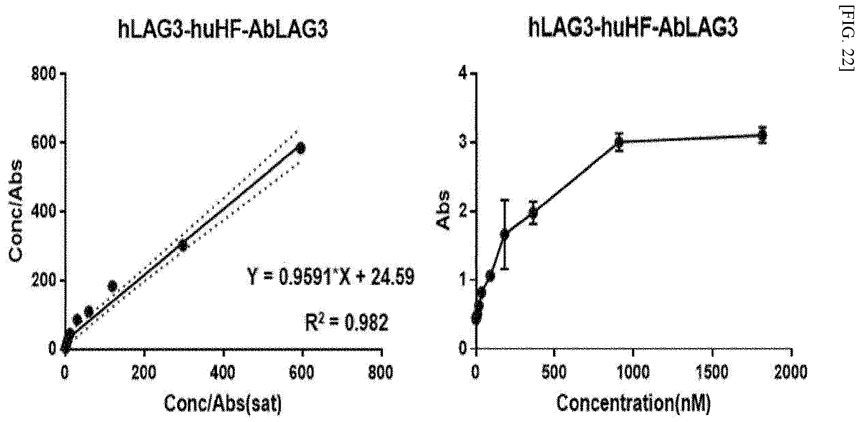

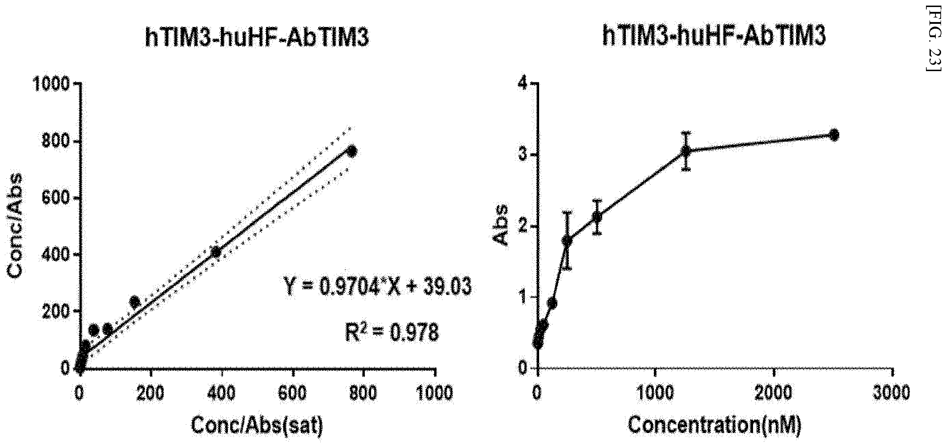

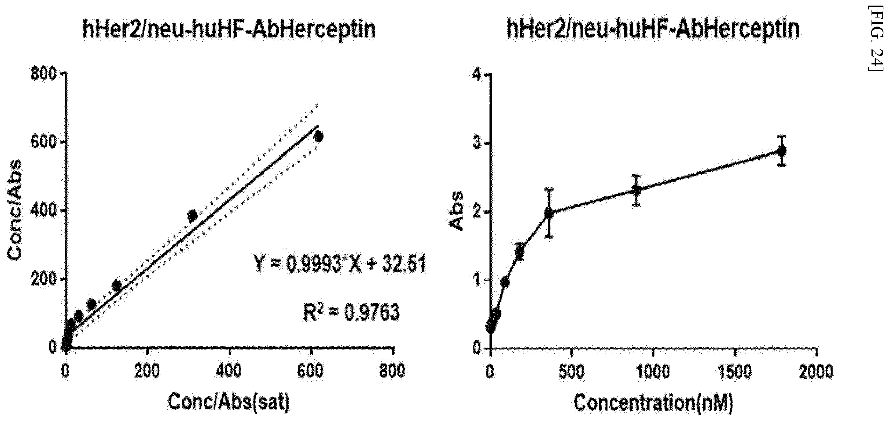

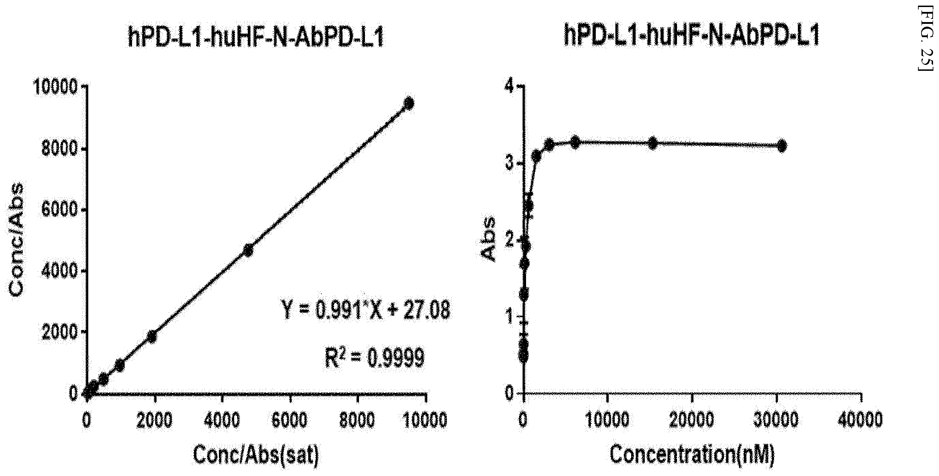

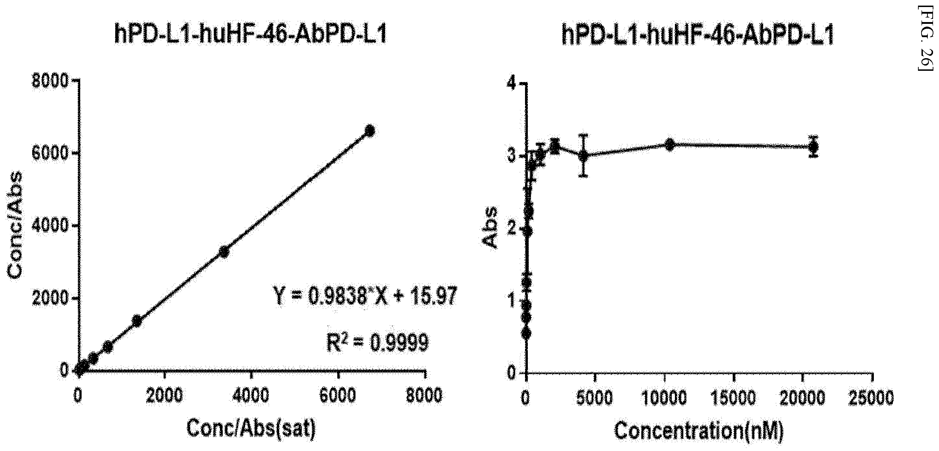

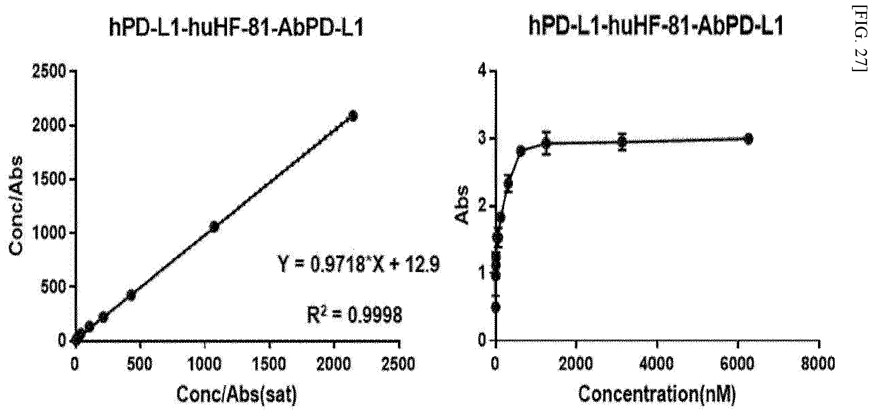

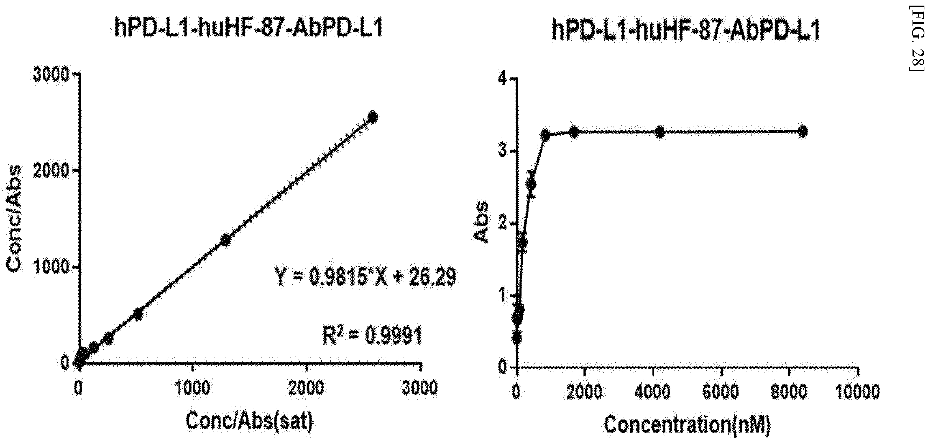

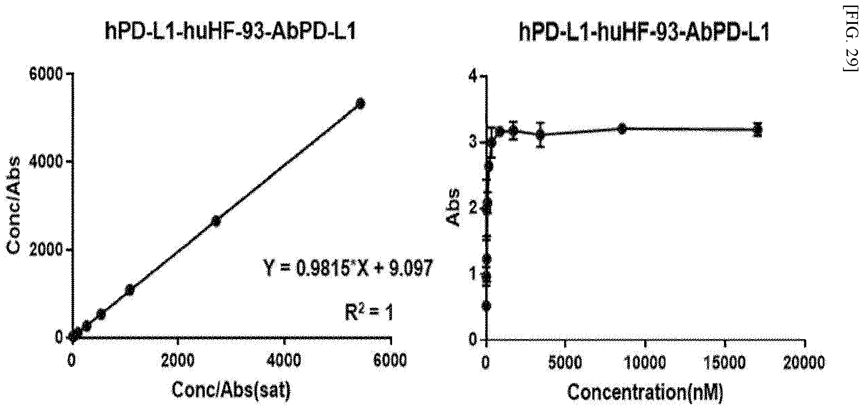

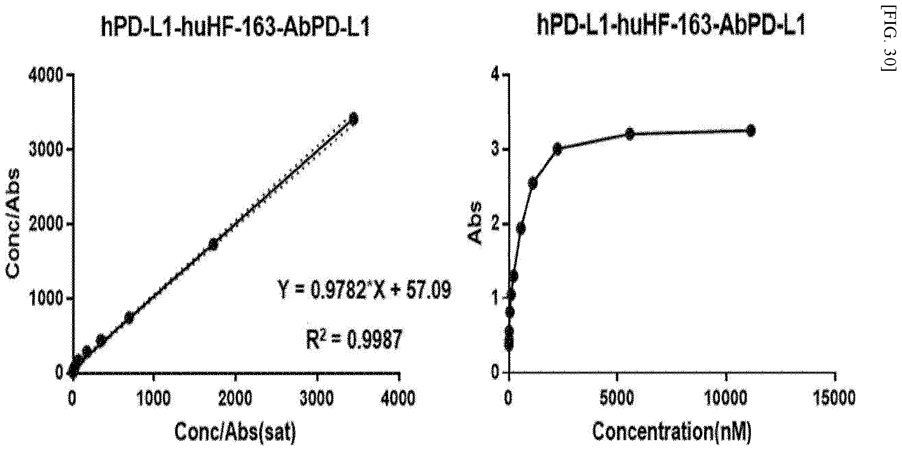

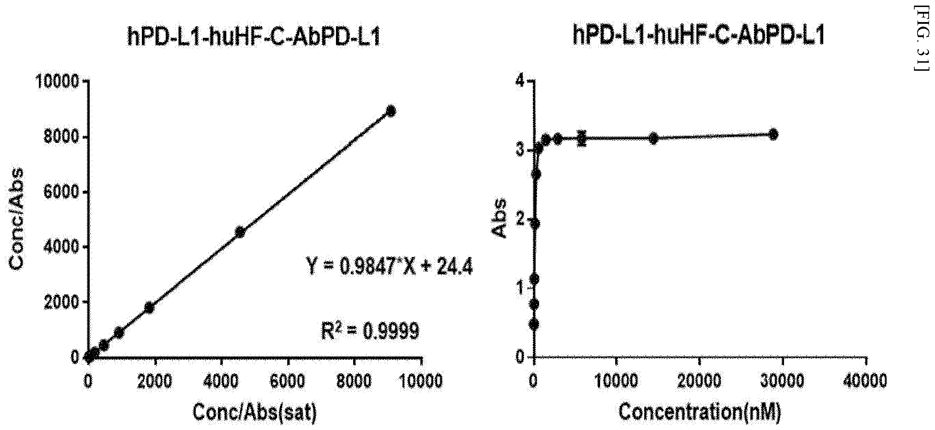

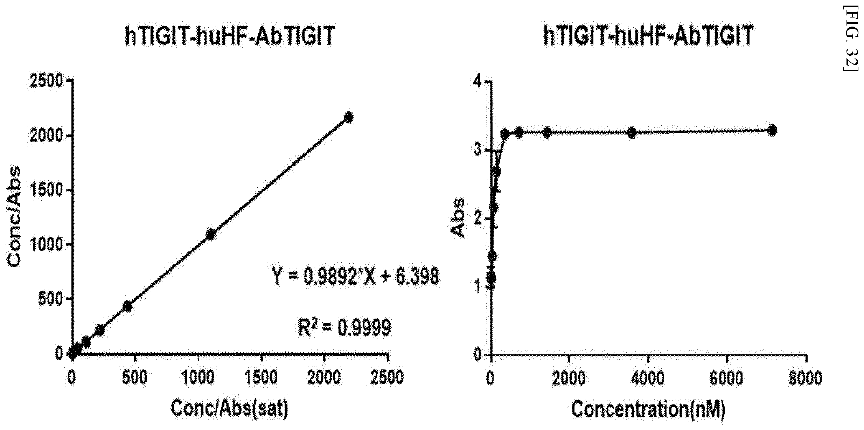

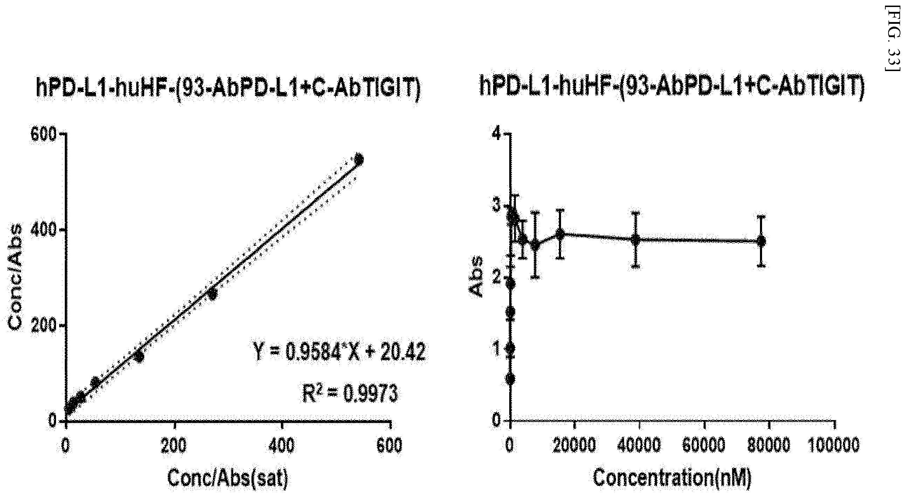

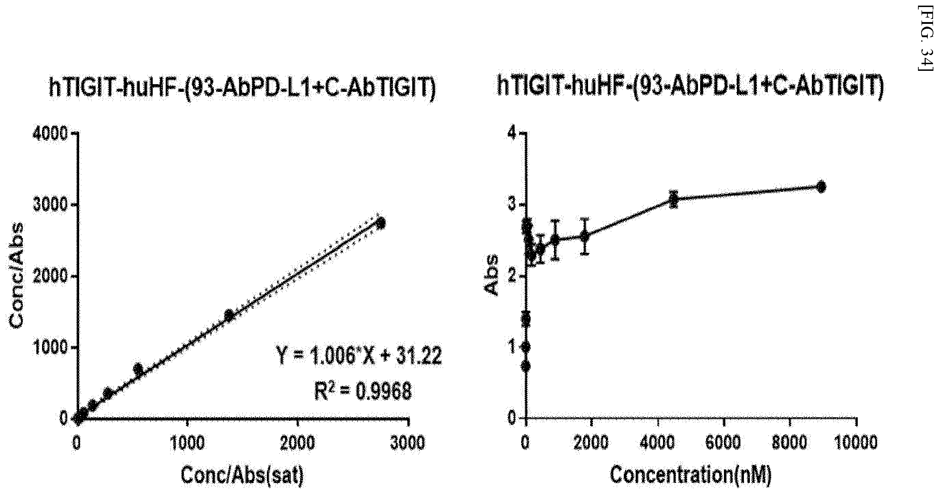

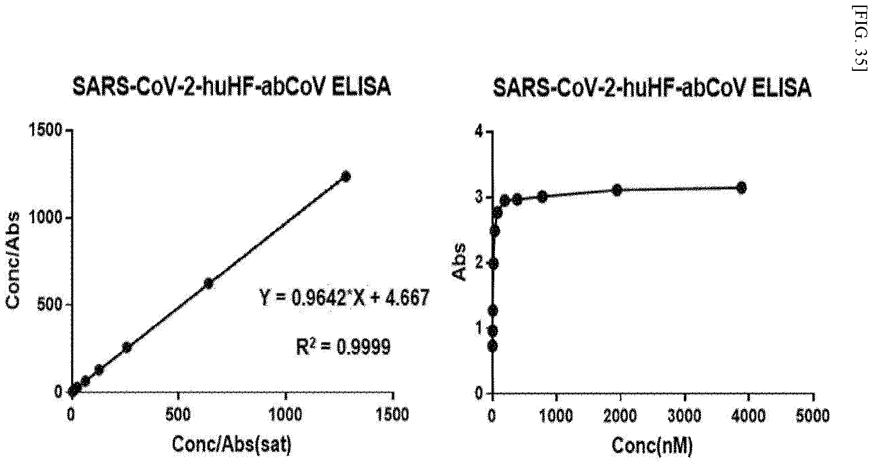

Measurement results are shown in Tables 6 to 9 below and FIGS. 14 to 35.

Table 6 shows the results of measuring the affinity of the self-assembly of 24 heavy chain CDR-ferritin monomers in which each antibody HCDR3 is fused at C-terminus, and Table 7 shows the results of measuring the affinity of the self-assembly of 24 heavy chain CDR-ferritin monomers in which each PD-L1 HCDR3 is fused at various positions, respectively.

Table 8 shows the results of measuring the affinity of each of: huHF-dual; a self-assembly of heavy chain CDR-ferritin monomer in which PD-L1 HCDR3 is fused to BC loop; a self-assembly of heavy chain CDR-ferritin monomer in which TIGIT HCDR3 is fused at C-terminus; and huHF-(93-AbPD) -L1+C-AbTIGIT), to each antigen.

Table 9 shows the results of measuring the affinity of huHF-dual to mouse antigen.

[TABLE 6]

| Section | Her2/neu (herceptin) | PD1 (keytruda) | PD-L1(tecentriq) | PD-L1 | TIM3 | LAG3 | TIGIT | SARS-Cov |
|---|---|---|---|---|---|---|---|---|
| C-term | 93.81 | 115.99 | 29.78(mouse)/ 47.92 (human) | 77.45 | 127.92 | 76.23 | 20.85 | 14.16 |

[TABLE 7]

| Section | PD-L1 |
|---|---|
| N-term | 87.16 |
| AB-loop | 49.28 |
| BC-loop(80) | 37.65 |
| BC-loop(86) | 85.43 |
| BC-loop(92) | 28.56 |
| DE-loop | 184.31 |
| C-term | 77.45 |

[TABLE 8]

| Section | hPD-L1 | hTIGIT |
|---|---|---|
| huHF-dual | 40.14 | 21.17 |
| huHF-93-AbPD-L1 | 28.56 | Not measured |
| huHF-C-AbTIGIT | Not measured | 20.85 |
| huHF-(93-AbPD-L1+C-AbTIGIT) | 58.29 | 101.44 |

[TABLE 9]

| Section | mPD-L1 | mTIGIT |
|---|---|---|
| huHF-dual | 27.17 | 42.37 |

**7. Use of a protein fused to a molecule that binds to an immune checkpoint molecule**

(1) Assessment of tumor inhibitory ability

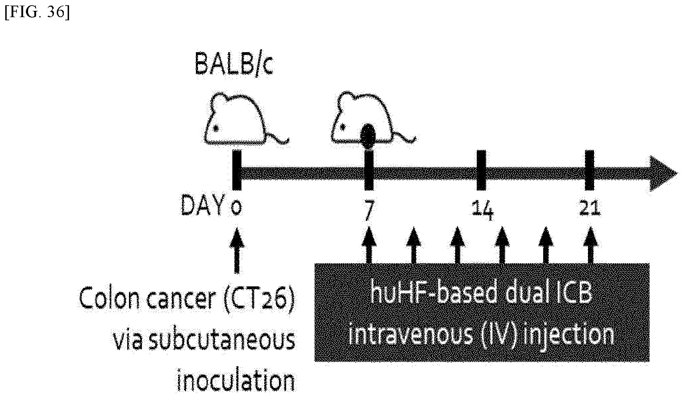

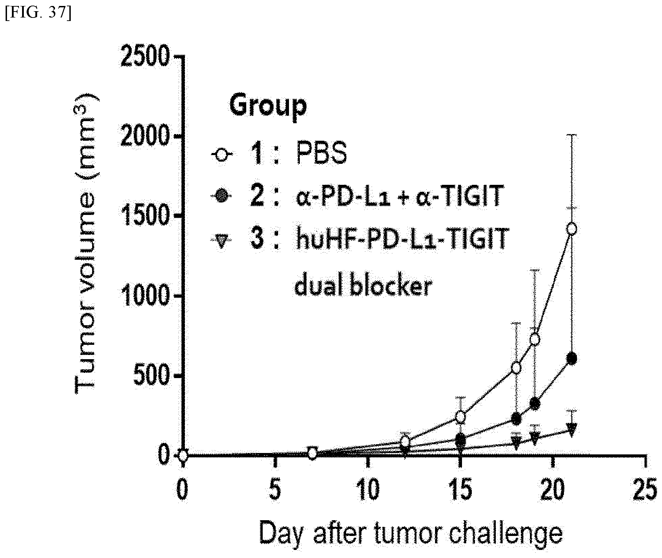

The tumor inhibitory ability of the protein was evaluated by subcutaneously inoculating a colorectal cancer cell line (CT26) into BALB/c mice and injecting the protein according to the schedule of FIG. 36 (FIG. 37).

Specifically, PBS, PD-L1 antibody, TIGIT antibody and huHF-PD-L1-TIGIT dual blocker protein, respectively, were intravenously injected into mice Balb/c having a certain size of colorectal cancer tumor (CT26) every 3 days. As a result of the observation, it could be seen that the huHF-PD-L1-TIGIT dual blocker protein showed similar tumor treatment efficacy to the antibody treatment. For the experiment, 4 mice per experimental group were used, and the size of cancer cells was calculated by Equation 1 below:

[Equation 1]

$$(\text{Tumor volume}) = (\text{Major axis}) \times (\text{Minor axis})^2 \times 0.52$$

In this regard, the experimental groups used herein were 1) PBS group, 2) antibody treatment group ($\alpha$-PD-L1, $\alpha$-TIGIT), and 3) protein treatment group (huHF-PD-L1-TIGIT dual blocker).

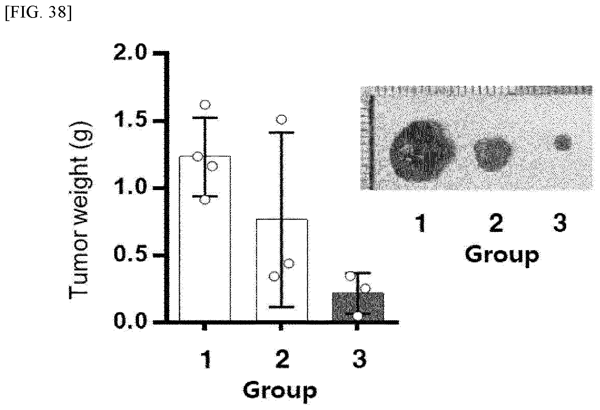

Further, the tumor tissue was extracted to measure the weight for each treatment group, and results thereof are shown in FIG. 38. From the results, it could be confirmed that the excellent anticancer efficacy of a protein in which molecules binding to PD-L1 and TIGIT have been fused.

(2) Efficiency analysis according to the site of fusion to ferritin monomer

A protein in which $\alpha$-PD-L1 HCDR3 is fused to different positions of the ferritin monomer was prepared in order to determine the tumor inhibitory ability.

Specifically, in order to compare the targeting efficiency of the FITC fluorescent substance-attached huHF-$\alpha$PD-L1 HCDR3 (AB, BC, CD, DE loops, C-terminus) protein against CT26 colorectal cancer, CT26 colorectal cancer cells were reacted with the protein nanoparticles at a concentration of 300 nM, followed by comparing the fluorescence signal in order to determine the cell uptake efficiency. As a result, it was confirmed that the huHF-$\alpha$PD-L1 HCDR3 (AB, BC, CD, DE loops, C-terminus) protein was bound to cancer cells and exhibited a fluorescence signal rather than the control huHF protein.

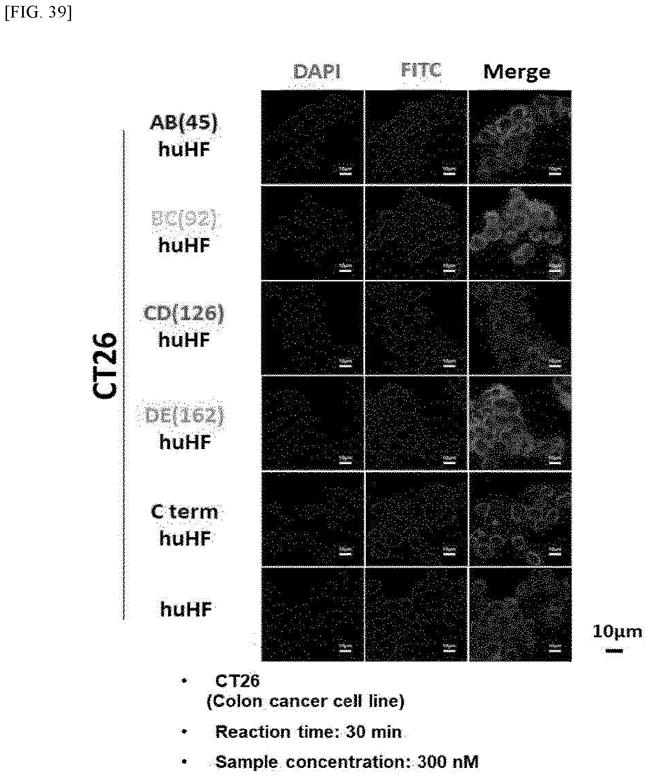

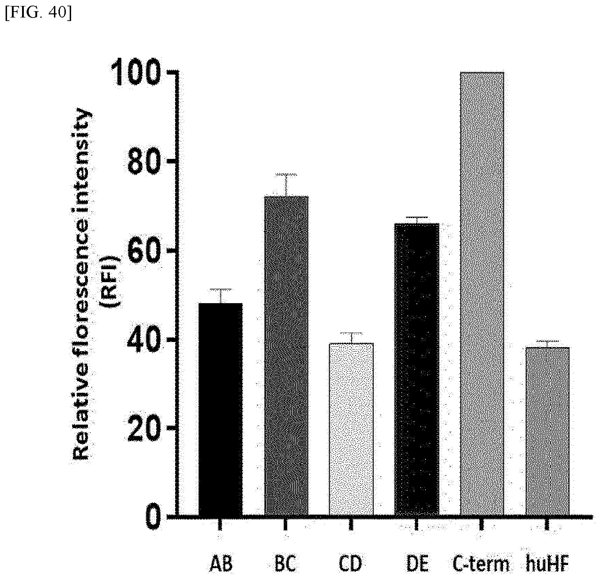

Results thereof are shown in FIG. 39, while the relative fluorescence intensity is shown in FIG. 40.

As a result of the investigation, it was confirmed that, regardless of the fusion site, it exhibited a stronger targeting ability compared to the antibody-like protein.

**8. Confirmation of affinity in cells of antibody-like proteins**

Target cells (immune cells, cancer cells) were cultured and aliquoted ($1*10^5$ pieces/10 ul, 1 FACS column (5 ml polystyrene round-bottom tube, Falcon (352052))).

Then, the cells were treated with the samples (drug, antibody (anti-mouse PD-L1 antibody (BE0101), anti-mouse TIGIT antibody (BE0274), anti-human PD-L1 (BE0285))) at 100 $\mu$l/FACS column, respectively, while the control group was treated with 100 $\mu$l of FACS buffer (0.1% BSA + 0.01% sodium azide in PBS) (3hr, room temperature).

After putting 1 ml of each FACS buffer into the FACS column, centrifugation was performed at 1700 rpm for 5 minutes followed by removing the supernatant, and this process was repeated 3 times.

Thereafter, the above cells were treated with fluorescence antibodies (anti his tag antibody-PE(SC-8036 PE), anti-IgG antibody-PE(PE goat F(ab')2 anti-rat (IgG) secondary antibody(ab7010), PE F(ab')) 2-goat anti-mouse IgG (H+L) secondary antibody (12-4010-82))), respectively (2hr, room temperature).

After putting 1 ml of each FACS buffer into the FACS column, centrifugation was performed at 1700 rpm for 5 minutes followed by removing the supernatant, and this process was repeated 3 times.

After putting 100 ul of FACS buffer in each sample FACS column, the sample was treated with 2 ul of 7-AAD (BD Pharmigen (559925)) for 5 minutes.

Analysis was performed using the ratio of target cells showing PE fluorescence among live cells in each FAC column by BD Biosciences and FACS equipment.

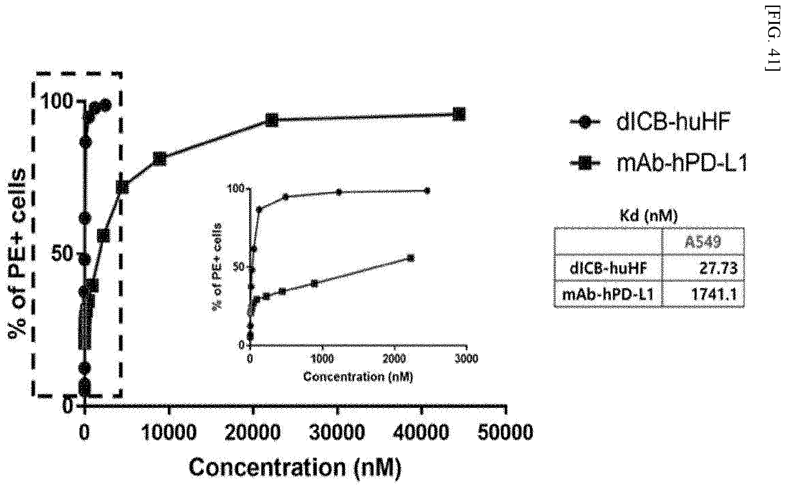

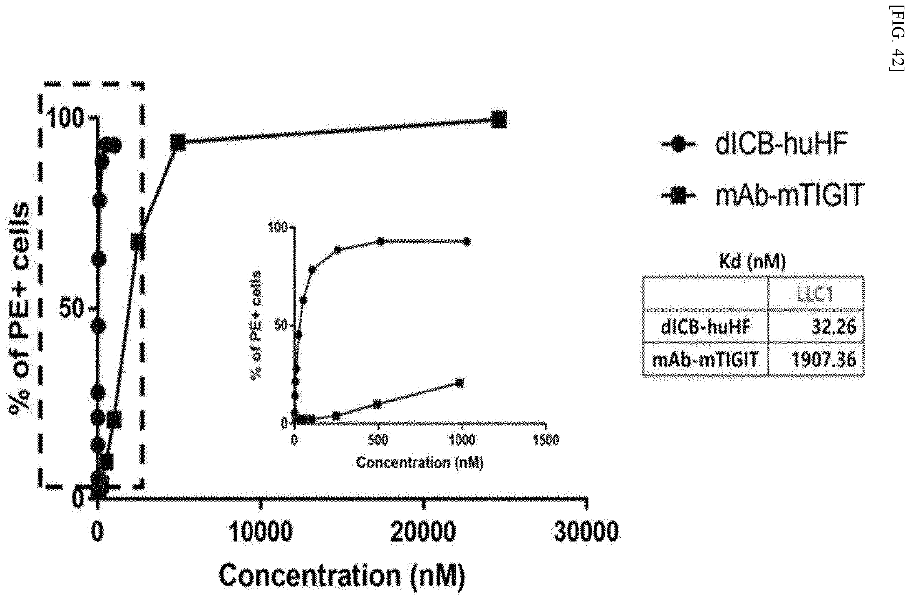

Measurement results are shown in FIGS. 41 and 42.

Referring to these figures, it could be confirmed that the antibody-like protein of the present invention exhibits very high avidity due to a significantly lower Kd value compared to the monoclonal antibody. This is because the antibody-like protein of the present invention has a plurality of CDRs, and thus can bind to a plurality of antigens.

**Claims**

1.  An antibody-like protein comprising self-assembly of a plurality of ferritin monomers, in which at least a CDR-ferritin monomer fused with a complementarity determining region is included.

2.  The antibody-like protein according to claim 1, wherein the complementarity determining region is any one selected from the group consisting of HCDR1, HCDR2, HCDR3, LCDR1, LCDR2, LCDR3, and conservative sequence variants thereof.

3. The antibody-like protein according to claim 1, wherein the complementarity determining region is exposed to a surface or outside of the protein for binding to an antigen.

4. The antibody-like protein according to claim 1, wherein the complementarity determining region has a length of 25aa or less.

5. The antibody-like protein according to claim 1, wherein the CDR-ferritin monomer is **characterized in that** the complementarity determining region is fused to a human ferritin heavy chain monomer.

6. The antibody-like protein according to claim 1, wherein the complementarity determining region is fused to any one selected from the group consisting of the inside of α-helix, between adjacent α-helices, N-terminus, C-terminus, A-B loop, B-C loop, C-D loop, D-E loop, between N-terminus and A helix, and between E helix and C-terminus of the ferritin monomer.

7. The antibody-like protein according to claim 1, wherein the CDR-ferritin monomer is **characterized in that** a plurality of complementarity determining regions of the antibody are fused to a single ferritin monomer.

8. The antibody-like protein according to claim 1, wherein the CDR-ferritin monomer is **characterized in that** at least one heavy chain complementarity determining region selected from the group consisting of HCDR1, HCDR2, HCDR3 and conservative sequence variants thereof of the antibody is fused to a single ferritin monomer.

9. The antibody-like protein according to claim 1, wherein the CDR-ferritin monomer is **characterized in that** at least one light chain complementarity determining region selected from the group consisting of LCDR1, LCDR2, LCDR3 and conservative sequence variants thereof of the antibody is fused to a single ferritin monomer.

10. The antibody-like protein according to claim 1, wherein the CDR-ferritin monomer comprises a heavy chain CDR-ferritin monomer to which at least one heavy chain complementarity determining region selected from the group consisting of HCDR1, HCDR2, HCDR3 and conservative sequence variants thereof of the antibody is fused, and a light chain CDR-ferritin monomer to which at least one light chain complementarity determining region selected from the group consisting of LCDR1, LCDR2, LCDR3 and conservative sequence variants thereof of the antibody is fused.

11. The antibody-like protein according to claim 1, wherein the complementarity determining region is fused to the DE loop or C-terminus of the CDR-ferritin monomer to form a four-fold symmetric structure.

12. The antibody-like protein according to claim 1, wherein the complementarity determining region is fused to the N-terminus, AB loop or BC loop of the CDR-ferritin monomer to form a two-fold symmetric structure.

13. The antibody-like protein according to claim 1, wherein the complementarity determining region is fused to the CD loop of the CDR-ferritin monomer to form a three-fold symmetric structure.

14. The antibody-like protein according to any one of claims 11 to 13, wherein the complementarity determining regions are disposed to be spaced apart from each other by a distance of 0.7 to 7 nm.

15. The antibody-like protein according to claim 1, wherein the CDR-ferritin monomer is **characterized in that** each complementarity determining region of two or more antibodies is fused to a single ferritin monomer.

16. The antibody-like protein according to claim 1, wherein the CDR-ferritin monomer comprises a first CDR-ferritin monomer to which at least one complementarity determining region of a first antibody is fused, and a second CDR-ferritin monomer to which at least one complementarity determining region of a second antibody is fused.

17. The antibody-like protein according to claim 16, wherein the first CDR-ferritin monomer comprises a first heavy chain CDR-ferritin monomer to which at least one heavy chain complementarity determining region selected from the group consisting of HCDR1, HCDR2, HCDR3 and conservative sequence variants thereof of the first antibody is fused, and a first light chain CDR-ferritin monomer to which at least one light chain complementarity determining region selected from the group consisting of LCDR1, LCDR2, LCDR3 and conservative sequence variants thereof of the first antibody is fused.

18. The antibody-like protein according to claim 16, wherein the second CDR-ferritin monomer comprises a second heavy chain CDR-ferritin monomer to which at least one heavy chain complementarity determining region selected from the group consisting of HCDR1, HCDR2, HCDR3 and conservative sequence variants thereof of the second antibody is fused, and a second light chain CDR-ferritin monomer to which at least one light chain complementarity determining region selected from the group consisting of LCDR1, LCDR2, LCDR3 and conservative sequence variants thereof of the second antibody is fused.

19. The antibody-like protein according to claim 16, wherein the first CDR-ferritin monomer and the second CDR-ferritin monomer are included in a ratio of 1:1 to 1:5.

20. The antibody-like protein according to claim 1, wherein the CDR-ferritin monomer comprises at least a first CDR-ferritin monomer fused with a plurality of complementarity determining regions of a first antibody group including a plurality of different antibodies, and a second CDR-ferritin monomer fused with a plurality of complementarity determining regions of a second antibody group including a plurality of different antibodies.

21. The antibody-like protein according to claim 1, wherein the plurality of ferritin monomers include at least a ferritin monomer to which the complementarity determining region is not fused.

22. The antibody-like protein according to claim 1, wherein the protein is formed by self-assembly of 24 CDR-ferritin monomers.

23. The antibody-like protein according to claim 1, wherein the protein has a spherical shape with a particle diameter of 8 to 50 nm.

24. The antibody-like protein according to claim 1, wherein the affinity (Kd) to the antigen bound to the complementarity determining region is 1000 nM or less.

25. The antibody-like protein according to claim 1, wherein the antibody is an antibody against any one selected from the group consisting of PD-1, Her-2/neu, VISTA, 4-1BBL, CD48, Galectin-9, Adenosine A2a receptor, CD80, CD86, ICOS, ICOSL, BTLA, OX-40L, CD155, BCL2, MYC, PP2A, BRD1, BRD2, BRD3, BRD4, BRDT, CBP, E2F1, MDM2, MDMX, PPP2CA, PPM1D, STAT3, IDH1, PD-L1, PD-L2, CD40L, LAG3, TIM3, TIGIT, BTLA, CD52, SLAMF7, 4-1BB, OX-40, ICOS, GITR, CD27, CD28, CD16, CD3, CD20, EGFR family, AXL, CSF1R, DDR1, DDR2, EPH receptor family, FGFR family, VEGFR family, IGF1R, LTK, PDGFR family, RET, KIT, KRAS, NTRK1, NTRK2 and SARS-Cov.

26. The antibody-like protein according to claim 1, wherein the antibody is an antibody against any one selected from the group consisting of gp100, MART-1, Melna-A, MAGE-A3, MAGE-C2, Mammaglobin-A, proteinsase-3, mucin-1, HPV E6, LMP2, PSMA, GD2, hTERT, PAP, ERG, NA17, ALK, GM3, EPhA2, NA17-A, TRP-1, TRP-2, NY-ESO-1, CEA, CA 125, AFP, Survivin, AH1, ras, G17DT, MUC1, Her-2/neu, E75, p53, PSA, HCG, PRAME, WT1, URLC10, VEGFR1, VEGFR2, E7, Tyrosinase peptide, B16F10, EL4 and neoantigen.

27. The antibody-like protein according to claim 1, wherein the antibody is an antibody against an antigen of a cancer selected from the group consisting of brain cancer, head and neck cancer, bladder cancer, breast cancer, cervical cancer, colon cancer, colorectal cancer, endometrial cancer, esophageal cancer, leukemia, lung cancer, liver cancer, ovarian cancer, pancreatic cancer, prostate cancer, rectal cancer, kidney cancer, gastric cancer, testicular cancer, uterine cancer, vascular tumor, squamous cell carcinoma, adenocarcinoma, small cell carcinoma, melanoma, glioma, neuroblastoma, sarcoma, laryngeal cancer, parotid cancer, biliary tract cancer, thyroid cancer, actinic keratosis, acute lymphocytic leukemia, acute myeloid leukemia, adenoid cystic carcinoma, adenoma, adenoid squamous cell carcinoma, anal canal cancer, anal cancer, anorectal cancer, astrocytoma, ganglion adenocarcinoma, basal cell carcinoma, bile cancer, bone cancer, bone marrow cancer, bronchial cancer, bronchial carcinoma, carcinoid, cholangiocarcinoma, chronic lymphocytic leukemia, chronic myelogenous leukemia, clear cell carcinoma, connective tissue cancer, cystic adenoma, digestive system cancer, duodenal cancer, endocrine system cancer, endoderm sinus tumor, endometrial hyperplasia, endometrial adenocarcinoma, endothelial cell carcinoma, ependymal cell, epithelial cell carcinoma, orbital cancer, focal nodular hyperplasia, gallbladder cancer, palpebral cancer, gastrobasal cancer, gastrinoma, glioblastoma, glucagonoma, heart cancer, hemangioblastoma, hemangioendothelioma, hemangioma, hepatodenoma, liver adenoma, hepatobiliary cancer, hepatocellular carcinoma, Hodgkin's disease, ileal cancer, insulinoma, intraepithelial neoplasia, intraepithelial squamous cell neoplasia, intrahepatic biliary tract cancer, invasive squamous cell carcinoma, jejunum cancer, joint cancer, pelvic cancer, giant cell carcinoma, colon cancer, lymphoma, malignant mesothelial cell tumor, medulloblastoma, medullary epithelioma, meningeal cancer, mesothelial cancer, metastatic carcinoma, oral cancer, mucoepithelial carcinoma, multiple myeloma, muscle cancer, nasal duct cancer, nervous system cancer, non-epithelial skin cancer, non-Hodgkin's lymphoma, soft cell carcinoma, oligodendroglioma, oral cancer, osteosarcoma, papillary serous adenocarcinoma, penile cancer, pharyngeal cancer, pituitary tumor, plasmacytoma, pseudosarcoma, pulmonary blastoma, rectal cancer, renal cell carcinoma, respiratory system cancer, retina blastoma, serous carcinoma, sinus cancer, skin cancer, small cell carcinoma, small intestine cancer, smooth muscle cancer, soft tissue cancer, somatostatin-secreting tumor, spine cancer, squamous cell carcinoma, striatal muscle cancer, submesothelial carcinoma, T cell leukemia, tongue cancer, ureter cancer, urethral cancer, cervical cancer, uterine trunk cancer, vaginal cancer, VIPoma, vulvar cancer, highly differentiated carcinoma, and Wilm's tumor.

28. The antibody-like protein according to claim 1, wherein the antibody is an antibody against an infectious disease antigen.

29. The antibody-like protein according to claim 28, wherein the infectious disease is a bacterial, fungal, viral, parasitic or prion-induced disease.

30. A pharmaceutical composition for treatment or prevention of a disease, comprising the antibody-like protein according to claim 1.

31. A composition for diagnosis of a disease, comprising the antibody-like protein according to claim 1.

32. A method for detecting an antigen, comprising treating an antigen with the antibody-like protein according to claim 1.

[FIG. 1]

[FIG. 2a]

[

FIG. 2b]

| Loop | distance (nm) |
|---|---|
| N-term | 5.945 |
| AB-loop(46) | 3.165 |
| BC-loop(81) | 2.993 |
| BC-loop(87) | 0.855 |
| BC-loop(93) | 3.971 |
| CD-loop(127) | 1.774 |
| DE-loop(163) | 1.47 |
| C-term | 1.383 |

[FIG. 3]

[FIG. 4]

23.279 kDa

[FIG. 5]

23.4 kDa

[FIG. 6]

22.972 kDa

[FIG. 7]

24.242 kDa

[FIG. 8]

23.586 kDa

[FIG. 9]

22.986 kDa

[FIG. 10]

26.137 kDa

[FIG. 11]

[FIG. 12]

[FIG. 13]

[FIG. 14]

mTIGIT-huHF-dual ELISA

mPD-L1-huHF-dual ELISA

hPD-L1-huHF-dual ELISA

hTIGIT-huHF-dual ELISA

[FIG. 15]

EP 4 238 984 A1

[FIG. 16]

[FIG. 17]

hTIGIT-huHF-dual ELISA

Y = 0.9808*X + 6.831

$R^2 = 1$

hPD-L1-huHF-dual ELISA

Y = 0.9662*X + 12.63

$R^2 = 0.9999$

[FIG. 18]

[FIG. 19]

hHer2/neu-huHF-AbHerceptin

hHer2/neu-huHF-AbHerceptin

Y = 0.9993*X + 32.51

$R^2$ = 0.9763

[FIG. 20]

EP 4 238 984 A1

[FIG. 21]

EP 4 238 984 A1

hPD1-huHF-AbKeytruda

$Y = 0.9655 \cdot X + 35.6$

$R^2 = 0.9556$

hPD1-huHF-AbKeytruda

[FIG. 22]

[FIG. 23]

hHer2/neu-huHF-AbHerceptin

$Y = 0.9993*X + 32.51$

$R^2 = 0.9763$

hHer2/neu-huHF-AbHerceptin

[FIG. 24]

EP 4 238 984 A1

[FIG. 25]

[FIG. 26]

EP 4 238 984 A1

hPD-L1-huHF-46-AbPD-L1

Y = 0.9838*X + 15.97

R² = 0.9999

hPD-L1-huHF-46-AbPD-L1

[FIG. 27]

[FIG. 28]

hPD-L1-huHF-87-AbPD-L1

Abs

Concentration(nM)

hPD-L1-huHF-87-AbPD-L1

$Y = 0.9815^*X + 26.29$

$R^2 = 0.9991$

Conc/Abs

Conc/Abs(sat)

[FIG. 29]

[FIG. 30]

[FIG. 31]

EP 4 238 984 A1

hPD-L1-huHF-C-AbPD-L1

$Y = 0.9847*X + 24.4$

$R^2 = 0.9999$

hPD-L1-huHF-C-AbPD-L1

[FIG. 32]

hPD-L1-huHF-(93-AbPD-L1+C-AbTIGIT)

hPD-L1-huHF-(93-AbPD-L1+C-AbTIGIT)

$Y = 0.9584^*X + 20.42$

$R^2 = 0.9973$

[FIG. 33]

EP 4 238 984 A1

[FIG. 34]

[FIG. 35]

SARS-CoV-2-huHF-abCoV ELISA

$Y = 0.9642^*X + 4.667$

$R^2 = 0.9999$

SARS-CoV-2-huHF-abCoV ELISA

[FIG. 36]

[FIG. 37]

[FIG. 38]

[FIG. 39]

- CT26
  (Colon cancer cell line)
- Reaction time: 30 min
- Sample concentration: 300 nM

[FIG. 40]

[FIG. 41]

[FIG. 42]

## INTERNATIONAL SEARCH REPORT

| International application No. |
| --- |
| **PCT/KR2021/015602** |

| A. | CLASSIFICATION OF SUBJECT MATTER |
| --- | --- |

**C07K 14/475**(2006.01)i; **C07K 16/28**(2006.01)i; **C07K 16/18**(2006.01)i; **A61P 35/00**(2006.01)i; **A61K 38/00**(2006.01)i

According to International Patent Classification (IPC) or to both national classification and IPC

| B. | FIELDS SEARCHED |
| --- | --- |

Minimum documentation searched (classification system followed by classification symbols)

C07K 14/475(2006.01); A61K 31/69(2006.01); A61K 38/17(2006.01); A61K 9/16(2006.01); A61K 9/51(2006.01); C07K 19/00(2006.01); C12N 15/62(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 페리틴(ferritin), 상보성 결정 영역(complementarity determining region), 융합(fusion), 항체(antibody), 결합력(affinity), 항체 유사 단백질(Antibody Like Protein, ALP)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | KR 10-1477123 B1 (KOREA RESEARCH INSTITUTE OF BIOSCIENCE AND BIOTECHNOLOGY et al.) 29 December 2014 (2014-12-29)<br>See abstract; and claims 1-31. | 1-32 |
| A | KADONOSONO, T. et al. Design strategy to create antibody mimetics harbouring immobilised complementarity determining region peptides for practical use. Scientific reports. 21 January 2020, vol. 10, thesis no.: 891, pp. 1-11.<br>See abstract; figure 1; and pages 5-7. | 1-32 |
| A | KR 10-2018-0008353 A (KOREA INSTITUTE OF SCIENCE AND TECHNOLOGY) 24 January 2018 (2018-01-24)<br>See abstract; and claims 1-28. | 1-32 |
| A | KR 10-2015-0088597 A (UNIST ACADEMY-INDUSTRY RESEARCH CORPORATION) 03 August 2015 (2015-08-03)<br>See abstract; and claims 1-20. | 1-32 |

☑ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **25 February 2022** | **25 February 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2021/015602** |

## C. DOCUMENTS CONSIDERED TO BE RELEVANT

| Category* | Citation of document, with indication, where appropriate, of the relevant passages | Relevant to claim No. |
| --- | --- | --- |
| A | US 6207804 B1 (HUSTON, J. S. et al.) 27 March 2001 (2001-03-27)<br>    See entire document. | 1-32 |

Form PCT/ISA/210 (second sheet) (July 2019)

**INTERNATIONAL SEARCH REPORT**

International application No.

**PCT/KR2021/015602**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
Information on patent family members

International application No.

**PCT/KR2021/015602**

| Patent document cited in search report | | | Publication date (day/month/year) | Patent family member(s) | | | Publication date (day/month/year) |
|---|---|---|---|---|---|---|---|
| KR | 10-1477123 | B1 | 29 December 2014 | KR | 10-2013-0039672 | A | 22 April 2013 |
| | | | | WO | 2013-055058 | A2 | 18 April 2013 |
| | | | | WO | 2013-055058 | A3 | 01 August 2013 |
| | | | | WO | 2013-055058 | A9 | 13 June 2013 |
| KR | 10-2018-0008353 | A | 24 January 2018 | CA | 3040440 | A1 | 18 January 2018 |
| | | | | CN | 109803642 | A | 24 May 2019 |
| | | | | EP | 3501509 | A1 | 26 June 2019 |
| | | | | KR | 10-1953617 | B1 | 23 May 2019 |
| | | | | US | 10905774 | B2 | 02 February 2021 |
| | | | | US | 2019-0216947 | A1 | 18 July 2019 |
| | | | | WO | 2018-012952 | A1 | 18 January 2018 |
| KR | 10-2015-0088597 | A | 03 August 2015 | KR | 10-1630858 | B1 | 15 June 2016 |
| US | 6207804 | B1 | 27 March 2001 | AT | 120761 | T | 15 April 1995 |
| | | | | AT | 243754 | T | 15 July 2003 |
| | | | | AT | 295420 | T | 15 May 2005 |
| | | | | AT | 419355 | T | 15 January 2009 |
| | | | | AT | 503496 | T | 15 April 2011 |
| | | | | AU | 1804988 | A | 21 December 1988 |
| | | | | AU | 3612293 | A | 03 September 1993 |
| | | | | AU | 612370 | B2 | 11 July 1991 |
| | | | | AU | 6280201 | A | 24 December 2001 |
| | | | | AU | 648591 | B2 | 28 April 1994 |
| | | | | AU | 675929 | B2 | 27 February 1997 |
| | | | | AU | 8579991 | A | 13 February 1992 |
| | | | | CA | 1341415 | C | 07 January 2003 |
| | | | | CA | 1341614 | C | 24 May 2011 |
| | | | | CA | 2129663 | A1 | 19 August 1993 |
| | | | | CA | 2129663 | C | 05 July 2005 |
| | | | | CA | 2372813 | A1 | 19 August 1993 |
| | | | | CA | 567480 | C | 07 January 2003 |
| | | | | CA | 617156 | C | 24 May 2011 |
| | | | | CN | 1317661 | C | 23 May 2007 |
| | | | | CN | 1592903 | A | 09 March 2005 |
| | | | | DE | 3853515 | T2 | 17 August 1995 |
| | | | | DE | 3853515 | T3 | 25 August 2005 |
| | | | | DE | 3856559 | T2 | 29 April 2004 |
| | | | | DE | 69333807 | T2 | 02 February 2006 |
| | | | | EP | 0318554 | A1 | 07 June 1989 |
| | | | | EP | 0318554 | B1 | 05 April 1995 |
| | | | | EP | 0318554 | B2 | 12 January 2005 |
| | | | | EP | 0623679 | A1 | 09 November 1994 |
| | | | | EP | 0623679 | B1 | 25 June 2003 |
| | | | | EP | 0625200 | A1 | 23 November 1994 |
| | | | | EP | 0625200 | B1 | 11 May 2005 |
| | | | | EP | 1514934 | A2 | 16 March 2005 |
| | | | | EP | 1514934 | A3 | 19 April 2006 |
| | | | | EP | 1514934 | B1 | 31 December 2008 |
| | | | | EP | 1573415 | A2 | 14 September 2005 |
| | | | | EP | 1997894 | A2 | 03 December 2008 |
| | | | | EP | 1997894 | A3 | 04 November 2009 |

Form PCT/ISA/210 (patent family annex) (July 2019)

## INTERNATIONAL SEARCH REPORT
### Information on patent family members

International application No.

**PCT/KR2021/015602**

| Patent document cited in search report | Publication date (day/month/year) | Patent family member(s) | | | Publication date (day/month/year) |
|---|---|---|---|---|---|
| | | EP | 1997894 | B1 | 30 March 2011 |
| | | JP | 02-500329 | A | 08 February 1990 |
| | | JP | 08-500962 | A | 06 February 1996 |
| | | JP | 2003-284577 | A | 07 October 2003 |
| | | JP | 2004-503854 | A | 05 February 2004 |
| | | JP | 2005-104965 | A | 21 April 2005 |
| | | JP | 2005-168489 | A | 30 June 2005 |
| | | JP | 2007-228979 | A | 13 September 2007 |
| | | JP | 2007-231027 | A | 13 September 2007 |
| | | JP | 2008-289500 | A | 04 December 2008 |
| | | JP | 2009-035558 | A | 19 February 2009 |
| | | JP | 4236493 | B2 | 11 March 2009 |
| | | KR | 10-0403714 | B1 | 01 November 2003 |
| | | KR | 10-2001-0112686 | A | 21 December 2001 |
| | | US | 2001-0056418 | A1 | 27 December 2001 |
| | | US | 2002-0168375 | A1 | 14 November 2002 |
| | | US | 2005-0058638 | A1 | 17 March 2005 |
| | | US | 2006-0147444 | A1 | 06 July 2006 |
| | | US | 2007-0031931 | A1 | 08 February 2007 |
| | | US | 5091513 | A | 25 February 1992 |
| | | US | 5132405 | A | 21 July 1992 |
| | | US | 5258498 | A | 02 November 1993 |
| | | US | 5476786 | A | 19 December 1995 |
| | | US | 5482858 | A | 09 January 1996 |
| | | US | 5534254 | A | 09 July 1996 |
| | | US | 5753204 | A | 19 May 1998 |
| | | US | 5837846 | A | 17 November 1998 |
| | | US | 5877305 | A | 02 March 1999 |
| | | US | 7099861 | B2 | 29 August 2006 |
| | | US | 7138497 | B2 | 21 November 2006 |
| | | WO | 01-96978 | A2 | 20 December 2001 |
| | | WO | 88-09344 | A1 | 01 December 1988 |
| | | WO | 93-16185 | A2 | 19 August 1993 |
| | | WO | 93-16185 | A3 | 30 September 1993 |

Form PCT/ISA/210 (patent family annex) (July 2019)